# EUROPEAN PATENT SPECIFICATION

(11) **EP 1 422 235 B1**
(45) Date of publication and mention of the grant of the patent: **04.10.2006**
(21) Application number: 02751191.4
(22) Date of filing: 24.07.2002
(51) Int. Cl.: C07J 5/00, C07J 71/00, C07J 75/00

(54) **STEREOSELECTIVE METHOD OF PRODUCING 6ALPHA-FLUOROPREGNANES AND INTERMEDIARIES**
STEREOSELEKTIVES VERFAHREN ZUR HERSTELLUNG VON 6ALPHA-FLUORPREGNANEN UND ZWISCHENPRODUKTEN
PROCEDE STEREOSELECTIF DE PRODUCTION DE 6ALPHA-FLUORPREGNANES ET D'INTERMEDIAIRES

(30) Priority: 26.07.2001 ES 200101754
(43) Date of publication of application: 26.05.2004
(73) Proprietor: Ragactives, S.L., 47151 Boecillo (Valladolid) (ES)
(72) Inventor: MURILLO GARRIDO, Jose Vicente, E-47151 Boecillo, Valladolid (ES); SILVA GUISASOLA, Luis Octavio, E-47151 Boecillo, Valladolid (ES); MARTIN JUAREZ, Jorge, E-47151 Boecillo, Valladolid (ES)
(74) Representative: Maldonado Jordan, Julia
(86) International application number: PCT/ES2002/000372
(87) International publication number: WO 2003/010181

(56) References cited:
- EP-A- 1 207 166
- ES-T3- 2 091 100
- FR-A- 2 002 495
- US-A- 4 036 831
- US-A- 5 086 190
- TAYLOR S.D. ET AL: 'Recent advances in electrophilic fluorination' TETRAHEDRON vol. 55, no. 43, 1999, pages 12431 - 12477, XP004179337
- THOMAS M.G. ET AL: 'The synthesis of A- and B-ring fluorinated analogues of cholesterol' J. CHEM. SOC., PERKIN TRANS. 1 vol. 21, 1999, pages 3191 - 3198, XP002976823
- PROCOPIOU P.A. ET AL: 'An extremely powerful activation reaction of alcohols with anhydrides catalyzed by trimethylsilyl trifluoromethanesulfonate' J. ORG. CHEM. vol. 63, no. 7, 1998, pages 2342 - 2347, XP002976824

## Description

### FIELD OF THE INVENTION

The invention refers to a process of high stereoselectivity for the production of 6α-fluorpregnanes, carried out through new 3-(trisubstituted)silyloxy-pregna-3,5-dienes and in which the fluorine atom is introduced by means of the use of a fluorinating agent of the N-fluorosulfonimide or N-fluorosulfonamide type. The 6α-fluorpregnanes obtained are useful as synthesis intermediates for obtaining steroids which have a therapeutic application as anti-inflammatory and anti-asthmatic agents.

### BACKGROUND OF THE INVENTION

The preparation of 6α-fluorinated steroids has been disclosed in numerous patents and publications. United States patents US 2,838,499 and US 3,499,016 disclose a process on a 3-keto-Δ⁴-steroid consisting of the activation of position 6 by formation of a ketal at 3 and shifting of the double bond to position 5,6, formation of the epoxide and opening thereof to form fluorohydrin (6β-fluor-5α-hydroxy). Deprotection of the ketone at 3 and the removal of the hydroxy group gives the 6β-fluor-3-keto-Δ⁴-steroid which is subsequently isomerized to the corresponding 6α-fluor derivative. Similar techniques have been used in patents US 3,014,938 and US 4,898,693.

US patent 3,178,412 discloses a process for the introduction of the fluorine atom at position 6, also using 3-keto-Δ⁴-steroids as substrates. Activation of position 6 occurs due to the formation of an enol ether at position 3 originating the shifting of the double bonds to positions 3,4 and 5,6. The intermediate formed reacts with perchloryl fluoride by introducing the fluorine at position 6β and restoring the 3-keto-Δ⁴ system.

US patent 3,506,650 discloses a similar process, but the substrate used is a 3-keto-Δ^{1,4} steroid. Activation of position 6 is achieved by the formation of an enol ether at position 3 and shifting of the double bonds, as in the previous case, the double bond at position 1,2 being maintained.

US patent 4,188,322 discloses a process for preparing 6-halo pregnanes functionalized with the β-epoxide group at positions 9,11. The activation of position 6 by the formation of enol acetate is disclosed using isopropenyl acetate as a reagent, and the introduction of the fluorine atom is disclosed using perchloryl fluoride.

Spanish patent ES 2,091,100 discloses a process for the preparation of 6α,9α-difluorinated steroid derivatives of androstane with an ester function at position 17. Even though the patent mentions several activating groups of position 6 (formation of enol esters and ethers) and different electrophilic fluorination reagents (N-fluoropyridinium salts, acetyl or trifluoroacetyl hypofluorite, N-fluorosulfonamides or N-fluorosulfonimides or N-fluoro-N-chloromethyl-triethylenediamine bis-tetrafluoroborate (Selectfluor^{R})), it only discloses in the examples the reaction through the formation of enol benzoates and electrophilic fluorination with Selectfluor^{R}.

Umemoto et al. (J. Am. Chem. Soc. 1990, 112, 8563 and J. Org. Chem. 1995, 60, 6565) disclose the formation of 17-hydroxy-6-fluor-androsta-4-ene-3-ones through the formation of enol acetates, enol ethers or silyl enol ethers and using N-fluoropyridinium salts as a fluorinating agent. The authors present results leading to mixtures of fluorinated products 4 and 6, the 4-fluorinated impurity in many cases being the majority. From the mixture of 6-fluorinated isomers, the 6β isomer is always obtained as a majority.

A.J. Poss et al. (J. Org. Chem. 1991, 56, 5962) disclose the formation of 6-fluor-17-acetoxy-androst-4-ene-3-ones and 6-fluor-pregna-4-ene-3,20-diones through the formation of enol acetates or trimethylsilyl enol esters and using N-fluoropyridinium heptafluoroborate (not a commercially attainable reagent) as an electrophilic fluorinating agent. Quite variable yields are obtained with α/β isomeric mixtures at variable ratios (from 6:1 to 1:3). In one of the cases, the exclusive obtainment of the α isomer with a 36% yield is disclosed. By varying the conditions to increase the yield, new isomeric mixtures are obtained.

The formation of 6-fluoro steroids through enol acetate and using Selectfluor® as a fluorinating reagent has been disclosed by Sankar (J. Org. Chem. 1993, 58, 2791). The 4-fluorinated derivative is not obtained in the conditions used, although 6α/6β epimeric mixtures at similar ratios are always obtained, or the β isomer is obtained as the majority.

Harrington et al. (Org. Process and Development, 1997, 1, 217) carries out a review of different fluorinating reagents for the fluorination at position 6 of enol acetates of androstenediones, androstadienediones or 17-acetoxyandrostenediones. The use of N-fluorobenzenesulfonimide leads to a 95:5 ratio of the isomeric mixture at 6 in favor of the beta isomer. The use of other fluorinating reagents, such as N-fluoropyridinium heptafluoroborate or Selectfluor® leads to epimeric mixtures at ratios from 9:1 (in favor of the alpha isomer) to 0.8:1, noticeable amounts of the 3-keto-4,6-dione impurity being obtained. The authors conclude that the reagent giving the best yields is Selectfluor®, although practically without stereoselectivity.

A.J. Poss (Tetrahedron Letters 1999, 40, 2673) uses 1-fluoro-4-hydroxy-1,4-diazoniabicyclo[2.2.2]octane bis(tetrafluoroborate) in the fluorination at position 6 of enol acetates or ethers of 21-hydroxyprogesterone. α/β epimeric ratios of 1:2.2 to 1:2.4 are obtained.

The use of N-fluorobenzenesulfonimide has been disclosed in the literature for the electrophilic fluorination of different substrates (Taylor et al., Tetrahedron 55 (1999) 12431), but it has never been satisfactorily used for the introduction of a fluorine atom at position 6α in steroids with high stereoselectivity.

It is known that the formation of hydrocarbonated ethers or enol esters at position 3 occurs with difficulty, generating the formation of byproducts and not very high yields, especially when working with 3-keto-Δ^{1,4} steroids, since the cross conjugation seems to hinder the formation of these enols. On the other hand, when isopropenyl acetate is used for the formation of enol acetates, the high reactivity of this compound causes the unwanted acetylation of hydroxyl groups which may be present in the molecule (for example, at position 17), as well as the dienone-phenol rearrangement, leading the obtainment of byproducts. Likewise, the reagents used for the formation of these enol esters or ethers can cause unwanted reactions if other functional groups, such as ketone groups, are present in the molecule.

It is also known that when the reagents described in the state of the art are used, the electrophilic fluorination step gives 6α/6β epimeric mixtures which, at times, are difficult to separate by industrial purification processes, such as crystallization with solvents. Reaction byproducts, such as 4-fluoro steroids, 3-keto-4,6-dienones, D-homo derivatives and 6-chloro steroids are also produced (when the reaction is carried out with perchloryl fluoride). All this generates the occurrence of impurities impossible to purify in the final pharmaceutical active ingredient and yield losses which act against the economy of the process.

On the other hand, perchloryl fluoride is a hugely toxic and hazardous gas and must be handled with great care in manufacturing plants, with the risks it implies for operators and property.

Therefore, there is still a need to find a process for the production of 6α-fluorpregnanes allowing an activation at position 6 of the steroid with high yields and using the electrophilic fluorination reaction with maximum stereoselectivity and with the minimum formation of byproducts, as well as using safe reagents.

### SUMMARY OF THE INVENTION

The invention faces the problem of providing a high stereoselectivity process for the synthesis of 6α-fluorpregnanes.

The solution provided by this invention is based on the fact that the inventors have observed that fluorination of 3-(trisubstituted)silyloxy-pregna-3,5-diene with an N-fluorosulfonimide or N-fluorosulfonamide-type fluorinating agent leads to the stereoselective introduction of fluorine at position 6 of the pregnane derivative, with a very high 6α/6β fluorine epimeric ratio, and with a very low production of impurities [see Examples 2-10 and compare with Reference Examples 1-7].

Therefore, an object of this invention is constituted of a stereoselective process for the production of 6α-fluorpregnanes comprising reacting a 3-(trisubstituted)silyloxy-pregna-3,5-diene with an N-fluorosulfonimide or N-fluorosulfonamide-type fluorinating agent.

An additional object of this invention is constituted of 3-(trisubstituted)silyloxy-pregna-3,5-dienes and their use in the stereoselective synthesis of 6α-fluorpregnane.

Another additional object of this invention is constituted of a process for the synthesis of said 3-(trisubstituted)silyloxy-pregna-3,5-dienes comprising reacting a pregnane derivative and a silyl(trisubstituted) trifluoromethanesulfonate.

Another additional object of this invention is constituted of a stereoselective process for the production of 6α-fluorpregnanes comprising the silylation of a pregnane derivative with silyl(trisubstituted) trifluoromethanesulfonate to obtain a 3 (trisubstituted)silyloxy-pregna-3,5-diene and the fluorination of this silylated derivative with an N-fluorosulfonimide or N-fluorosulfonamide-type fluorinating agent.

### DETAILED DESCRIPTION OF THE INVENTION

The invention provides a process for the production of a 6α-fluorpregnane, of general formula (I): 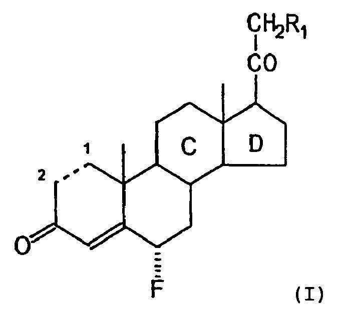 where
the dotted line between positions 1 and 2 represents a single or double bond;
R₁ is OH, OCOR₂, X, SO₃R₃ or an (R₇)(R₈)(R₉)SiO- group, where X is halogen, R₂ and R₃ are C₁₋₆ alkyl or phenyl optionally substituted by C₁₋₄ alkyl, and R₇, R₈ and R₉, equal or different, are C₁₋₆ alkyl or phenyl optionally substituted by C₁₋₄ alkyl;
the C ring of the steroid is: 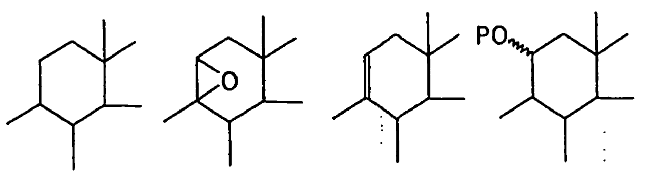
where
P is a protector group of the hydroxyl group; and
the D ring of the steroid is: 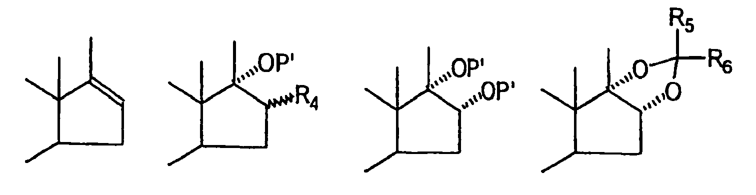
where
R₄ is H or CH₃ (α or β configuration);
R₅ and R₆, equal or different, are C₁₋₄ alkyl; and
each P', independently, is H, a protector group of the hydroxyl or an (R₇)(R₈)(R₉)Si-] group, where R₇, R₈ and R₉ have the previously mentioned meaning;
comprising reacting a 3-(trisubstituted)silyloxy-pregna-3,5-diene of general formula (IV): 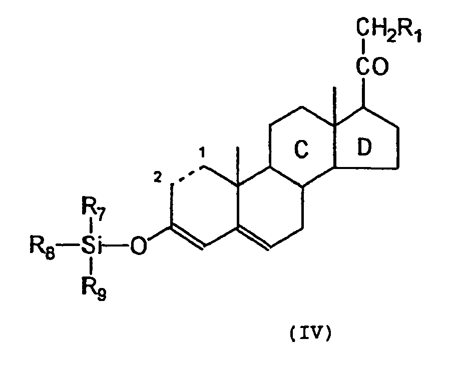 where
the dotted line between positions 1 and 2, R₁, R₇, R₈, R₉ and the C and D rings of the steroid, have the previously mentioned meaning,
with a fluorinating agent selected among:
(i) an N-fluorosulfonimide of general formula (V)

   R₁₀-O₂S-NF-SO₂-R₁₁ (V)

   where
   R₁₀ and R₁₁, equal or different, are C₁₋₄ alkyl with one or more hydrogen atoms optionally substituted by halogen, or phenyl optionally substituted by C₁₋₄ alkyl;
(ii) an N-fluorosulfonimide of general formula (VI) 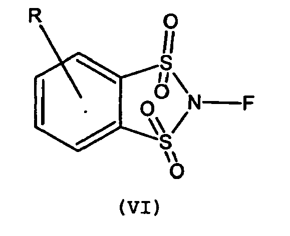 where
   R is a C₁₋₆ alkyl radical; and
(iii) an N-fluorosulfonamide of general formula (VII) 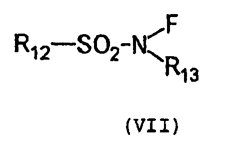 where
   R₁₂ is phenyl optionally substituted by C₁₋₄ alkyl; and
   R₁₃ is H, C₁₋₆ alkyl or phenyl optionally substituted by C₁₋₄ alkyl.

In the sense used in this description, the term C₁₋₄ or C₁₋₆ alkyl refers to a linear or branched radical derivative of an alkane, of 1 to 4 or of 1 to 6 carbon atoms, respectively.

As used in this description, the term "protector group of the hydroxyl" refers to a group capable of protecting the hydroxyl group or groups present in a compound such that the protected compound can be worked with without the occurrence of secondary reactions in which said hydroxyl groups would be involved if they were not protected, for example, alkanoyl, tetrahydropyranyl and benzyl.

A preferred class of compounds of formula (I) includes those compounds wherein the dotted line between positions 1 and 2 represents a double bond.

Another preferred class of compounds of formula (I) includes those compounds wherein R₁ is hydroxyl, acetate, pivalate, propionate, mesylate or chlorine.

Another preferred class of compounds of formula (I) includes those compounds presenting a 9β,11β-epoxy group in the C ring, or a double bond between positions 9 and 11 of the C ring.

Another preferred class of compounds of formula (I) includes those compounds wherein R₄ is αCH₃ or βCH₃.

Another preferred class of compounds of formula (I) includes those compounds containing an αOH group at position 17.

Another preferred class of compounds of formula (I) includes those compounds wherein R₅ and R₆ are, simultaneously, methyl.

A particularly preferred class of compounds of formula (I) includes those compounds wherein the dotted line between positions 1 and 2 represents a double bond, R₁ is hydroxyl, acetate, pivalate, propionate, mesylate or chlorine, they have a 9β,11β-epoxy group in the C ring, R₄ is αCH₃ or βCH₃, and they have an αOH group at position 17.

Another particularly preferred class of compounds of formula (I) includes those compounds wherein the dotted line between positions 1 and 2 represents a double bond, R₁ is hydroxyl, acetate, pivalate, propionate, mesylate or chlorine, they have a double bond between positions 9 and 11, R₄ is αCH₃ or βCH₃, and they have an αOH group at position 17.

The compounds of formula (I) can be used as synthesis intermediates of 6α-fluorinated steroids with pharmacological activity, such as Diflorasone, Flumethasone, Flunisolide, Fluocinolone Acetonide, Fluocinonide, Flurandrenolide, Fluticasone, Halobetasol, Fluocortolone, Diflucortolone, Paramethasone, etc., which are useful as anti-inflammatory and anti-asthmatic agents.

The reaction between the compound of formula (IV) and the electrophilic fluorinating agent or reagent [selected among the compounds of formula (V), (VI) and (VII)] can be carried out in a solvent compatible with the reagents used, i.e. it is inert against the reagents, preferably, in a halogenated organic solvent, such as methylene chloride, 1,2-dichloroethane or chloroform, although said reaction can also be carried out in other organic solvents such as aromatic hydrocarbons, acetonitrile or ethers. The fluorination reaction needs the presence of a base, preferably a weak, nitrogenated organic base such as triazole, aminotriazole, imidazole or pyridine. The reaction can be carried out at a temperature comprised between -40°C and +20°C, preferably between -10°C and 0°C.

Several tests carried out by the inventors have shown that the choice of the electrophilic fluorinating agent is key for stereoselectivity, for minimizing the formation of byproducts and for achieving the maximum yield of the reaction. The use of reagents of the N-fluoropyridinium salts, acetyl or trifluoroacetyl hypofluorite, N-fluoro-N-chloromethyl-triethylenediamine bis-tetrafluoroborate or perchloryl fluoride type leads to worse selectivity and a greater formation of byproducts than the use of N-fluorosulfonimides or N-fluorosulfonamides, as shown in this invention [see the comparative Examples included in this description as a reference]. With these reagents, and using the suitable substituents in the silyl group, a 6α/6β fluorine epimeric ratio of up to 90/1 and with a byproduct content of less than 5% altogether can be obtained. These results cannot be obtained with the experimental conditions disclosed in the state of the art.

The compounds of formulas (V), (VI) and (VII) are known and commercially attainable compounds, or they can be synthesized by means of methods disclosed in the state of the art (see US patent 5,478,964 and Davis et al. Tetrahedron Letters, 1991, 32, 1631-4).

The compounds of formula (IV) are new products, useful as intermediates in the stereoselective synthesis of 6α-fluorpregnanes and constitute an additional object of this invention. The compounds of formula (IV) can be obtained by means of a process comprising reacting a pregnane derivative of general formula (II) 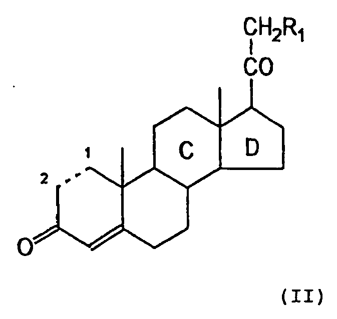 where
the dotted line between positions 1 and 2 represents a single or double bond;
R₁ is OH, OCOR₂, X, SO₃R₃, or an (R₇)(R₈)(R₉)SiO- group, where X is halogen, R₂ and R₃ are C₁₋₆ alkyl or phenyl optionally substituted by C₁₋₄ alkyl, and R₇, R₈ and R₉, equal_ or different, are C₁₋₆ alkyl or phenyl optionally substituted by C₁₋₄ alkyl;
the C ring of the steroid is: 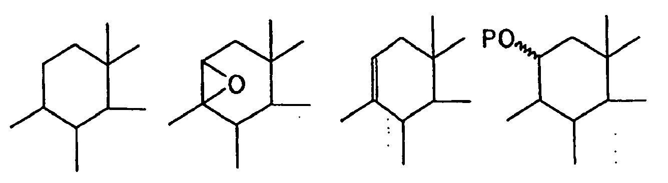
where
P is a protector group of the hydroxyl group; and
the D ring of the steroid is: 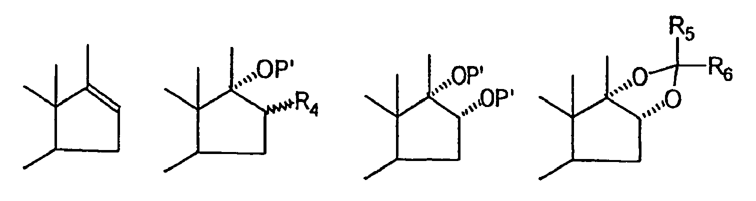
where
R₄ is H or CH₃ (α or β configuration);
R₅ and R₆, equal or different, are C₁₋₄ alkyl; and
each P', independently, is H, a protector group of the hydroxyl or an (R₇)(R₈)(R₉)Si- group, where R₇, R₈ and R₉ have the previously mentioned meaning;
with a (trisubstituted)silyl trifluoromethanesulfonate of general formula (III): 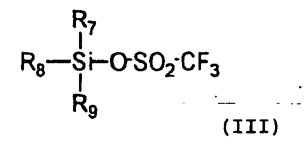 where
R₇, R₈ and R₉ have the previously mentioned meaning.

A preferred class of compounds of formula (IV) includes those compounds wherein the dotted line between positions 1 and 2 represents a double bond.

Another preferred class of compounds of formula (IV) includes those compounds wherein R₁ is acetate, pivalate, propionate or mesylate.

Another preferred class of compounds of formula (IV) includes those compounds presenting a 9β,11β-epoxy group in the C ring, or a double bond between positions 9 and 11 of the C ring.

Another preferred class of compounds of formula (IV) includes those compounds wherein R₄ is αCH₃ or βCH₃.

Another preferred class of compounds of formula (IV) includes those compounds containing an αOH group at position 17.

Another preferred class of compounds of formula (IV) includes those compounds wherein R₅ and R₆ are, simultaneously, methyl.

Another preferred class of compounds of formula (IV) includes those compounds wherein two groups selected among R₇, R₈ and R₉ are simultaneously methyl and the other one is t-butyl, or wherein R₇, R₈ and R₉ are simultaneously isopropyl.

A particularly preferred class of compounds of formula (IV) includes those compounds wherein the dotted line between positions 1 and 2 represents a double bond, R₁ is acetate, pivalate, propylate or mesylate, they have a 9β,11β-epoxy group in the C ring, R₄ is αCH₃ or βCH₃, they have an αOH group at position 17, two groups selected among R₇, R₈ and R₉ are simultaneously methyl and the other one is t-butyl, or R₇, R₈ and R₉ are simultaneously isopropyl.

Another particularly preferred class of compounds of formula (IV) includes those compounds wherein the dotted line between positions 1 and 2 represents a double bond, R₁ is acetate, pivalate, propionate or mesylate, they have a double bond between positions 9 and 11, R₄ is αCH₃ or βCH₃, they have an αOH group at position 17, two groups selected among R₇, R₈ and R₉ are simultaneously methyl and the other one is t-butyl, or R₇, R₈ and R₉ are simultaneously isopropyl.

The reaction between the compound of formula (II) and (III) can be carried out in an anhydrous medium, in a conventional solvent, preferably a halogenated solvent, such as dichloromethane or 1,2-dichloroethane, although other solvents can be used, such as acetonitrile or ethers, in the presence of a nitrogenated organic base, for example diisopropylethylamine, triethylamine, lutidine or collidine, preferably diisopropylethylamine, at a temperature comprised between -20°C and room temperature (15-25°C), preferably maintaining the temperature of the reaction between -10°C and 0°C.

Several tests carried out by the inventors have shown that the formation of silyl enol ether is very advantageous with regard to the formation of hydrocarbonated ethers or enol esters described in the state of the art, in reference to the minimization of byproducts and maximization of the yield of the reaction. The reagent used for the formation of silyl enol ethers is a trialkyl triflate or aryl silyl, preferably a trialkylsilyl triflate with long chain and/or branched hydrocarbonated residues, for example t-butyldimethyl or triisopropyl, since they give easily isolatable, crystalline compounds identifiable with conventional structural identification techniques. This type of substituents also provides excellent stereoselectivity in the fluorination reaction. The most preferable reagents are terc-butyldimethylsilyl triflate and triisopropylsilyl triflate, which are commercially attainable.

The compounds of formula (II) can have a functional free hydroxyl group at positions 16 and/or 21, which can be silylated by compound (III) to give a compound of formula (IV) disilylated or trisilylated at positions 3 and (16 and/or 21), which are also included within the scope of the compounds of formula (IV). These di- or trisilylated derivatives are obtained by silylation of compound (II) with hydroxy groups at positions 16 and/or 21 with a quantity of silylating reagent (compound (III)) at a compound (III):compound (II) molar ratio equal to or greater than 2 (to obtain the disilylated derivative) or equal to or greater than 3 (to obtain the trisilylated derivative). Alternatively, the di- or trisilylated derivatives of the compound of formula (IV) can be obtained by silylation of the mono- or disilylated compound (II) at positions 16 and/or 21 with said silylating reagent at the suitable ratio.

The invention also provides a process for the production of a 6α-fluorpregnane (I) comprising reacting said compound of formula (II) with said compound of formula (III) to obtain said compound of formula (IV), which subsequently reacts with an electrophilic fluorinating reagent of formula (V), (VI) or (VII) to obtain the compound of formula (I). The conditions for carrying out each one of the steps (silylation and fluorination) are those previously mentioned for each particular reaction. The intermediate (IV) obtained in the silylation step, if so desired, can be isolated by conventional methods (for example, by crystallization) or, if so desired, after the removal of water soluble contaminants, it can be used directly in the fluorination reaction. One advantage of the process for obtaining compound (I) by silylation of compound (II) and subsequent fluorination of the intermediate (IV), according to the invention, lies in the fact that the molar yield obtained in the transformation from compound (II) to compound (I) in some cases exceeds 75%, which is not achieved in the experimental conditions described in the state of the art.

The following examples illustrate the present invention, albeit without limiting it.

### Reference Example 1

### 6-fluor-9β,11β-epoxy-17,21-dihydroxy-pregna-1,4-diene-3,20-dione 17,21-diacetate

102 mL of isopropenyl acetate and 20.47 g of 9β,11β-epoxy-17,21-dihydroxy-pregna-1,4-diene-3,20-dione 21-acetate were mixed under inert atmosphere. The mixture was heated at 80°C and stirred for 3 hours at that temperature. Then, 5.1 g of potassium acetate were added, and the excess isopropenyl acetate was removed by vacuum distillation.

185 mL of absolute ethanol were added to the distillation residue and stirred until dissolution. Then, 15.35 g of anhydrous potassium acetate were added and the solution was inerted with a mild passage of nitrogen. The mixture was cooled to 0°C and 8.5 g of perchloryl fluoride were bubbled. Once the passage of gas was completed, the mixture was stirred at 0°C for 4 hours.

Then, the reaction mixture was added to 1.5 L of water pre-cooled at 5°C, and the resulting suspension was filtered. The product was vacuum dried, obtaining 23.97 g of a crude product which was analyzed by HPLC in the following conditions:
Detector: UV 254 nm
Flow rate: 1 mL/min
Eluent: Acetonitrile 45: Water 55
Column: Novapak C-18

A product with a 53% purity and a ratio of the following compounds were obtained:
- Isomer 6α of the titer: 79%
- Isomer 6β of the titer: 8%

### Reference Example 2

### A) 9β-11β-epoxy-3,17,21-trihydroxy-16β-methylpregna-1,3,5-triene-20-one 3,17,21-triacetate

400g of 9β,11β-epoxy-17α,21-dihydroxy-16β-methylpregna-1,4-diene-3,20-dione 21-acetate, 134.7 g of pyridinium p-toluenesulfonate and 4,000 mL of isopropenyl acetate were mixed at 20°C under inert atmosphere and heated under reflux. The reaction was maintained under reflux conditions for 6 hours. Once this was completed, it was cooled at 25°C and vacuum distilled, and the residue was used in the subsequent step.

### B) 6-fluoro-9β,11β-epoxy-17α,21-dihydroxy-16β-methylpregna-1,4-diene-3,20-dione 17,21-diacetate

The residue obtained in the previous step and 4,000 mL of acetonitrile were mixed under inert atmosphere, and the solution was cooled to at 0°C. Then, 436.9 g of N-fluoro-N-chloromethyl-triethylenediamine bis tetrafluoroborate (Selectfluor®) were slowly added. When the filling was completed, the suspension was maintained at 0°C for 1 hour.

Subsequently, 253 mL of 20% ammonia hydroxide were added, and the solution was vacuum distilled until removing the acetonitrile. 2,000 mL of ethyl acetate and 2,000 mL of water were added to the resulting residue, and this was then stirred. A sufficient quantity of 7% sodium bicarbonate was added until the pH was adjusted to 6.5 - 7. The organic phase was decanted and washed with 2,000 mL of water. The solution was vacuum distilled until obtaining an oil which was analyzed by HPLC in the following conditions:
Detector: UV 254 nm
Flow rate: 1 mL/min
Eluent: acetonitrile 45: water 55
Column: Nova-Pak® C₁₈

A ratio of the following compounds was obtained:
- Isomer 6α of the titer, 33%
- Isomer 6β of the titer, 37%

### Reference Example 3

### A) 9β,11β-epoxy-3-t-butyl-dimethylsilyloxy-17α,21-dihydroxy-16β-methylpregna-1,3,5-triene-20-one 21-acetate

10 g of 9β,11β-epoxy-17α,21-dihydroxy-16β-methylpregna-1,4-diene-3,20-dione 21-acetate, 6.1 mL of diisopropylethylamine and 100 mL of dichloromethane were mixed at 20°C under inert atmosphere at -4°C. Then, 7.49 mL of t-butyldimethylsilyl trifluoromethanesulfonate were slowly added. After the filling was completed, it was maintained at-4°C for 15 minutes. The temperature was increased to 10°C and 100 mL of water were added. The solution was stirred and decanted. The organic phase was vacuum distilled to obtain a residue.

### B) 6-fluoro-9β,11β-epoxy-17α,21-dihydroxy-16β-methylpregna-1,4-diene-3,20-dione 21-acetate

The residue obtained in the distillation was mixed with 100 mL of acetonitrile and cooled at 0°C under inert atmosphere. Then, 8.98 g of N-fluoro-N-chloromethyl-triethylenediamine bis tetrafluoroborate were slowly added. Once the filling was completed, the suspension was maintained for 1 hour and then precipitated on 200 mL of water and 5 mL of 20% ammonium hydroxide. It was filtered and washed. 7.2 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 49%
- Isomer 6β of the titer, 16%

### Reference Example 4

### A) 9β,11β-epoxy-3-ethoxy-17,21-dihydroxy-16β-methylpregna-1,3,5-triene-20-one 21-acetate

20 g of 9β,11β-epoxy-17α,21-dihydroxy-16β-methylpregna-1,4-diene-3,20-dione 21-acetate, 1.1 g of p-toluenesulfonic acid, 20 mL of triethyl orthoformate, 0.46 mL of pyridine and 100 mL of ethanol were mixed at 20°C under inert atmosphere. The reaction was maintained at this temperature, and once completed, was vacuum distilled to 60 mL, was stirred at 0°C for one hour, was filtered and washed. 10 g of the expected product were obtained.

### B) 6-fluoro-9β,11β-epoxy-17α,21-dihydroxy-16β-methylpregna-1,4-diene-3,20-dione 21-acetate

10g of 9β,11β-epoxy-3-ethoxy-17,21-dihydroxy-16β-methylpregna-1,3,5-triene-20-one 21-acetate and 100 mL of acetonitrile were mixed under inert atmosphere and were cooled at 0°C. Then, 8.4 g of N-fluoro-N-chloromethyl-triethylenediamine bis tetrafluoroborate were slowly added. Once the filling was completed, the suspension was maintained for 1 hour and then precipitated on 200 mL of water and 5 mL of 20% ammonium hydroxide. It was filtered and washed. 6.2 g of a product were obtained which were analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 72%
- Isomer 6β of the titer, 12%

### Reference Example 5

### A) 9β,11β-epoxy-3-t-butyl-dimethylsilyloxy-17α,21-dihydroxy-16β-methylpregna-1,3,5-triene-20-one 21-acetate

10g of 9β,11β-epoxy-17α,21-dihydroxy-16β-methylpregna-1,4-diene-3,20-dione 21-acetate, 6.1 mL of diisopropylethylamine and 100 mL of dichloromethane were mixed at 20°C under inert atmosphere at -4°C. Then, 7.49 mL of t-butyldimethylsilyl trifluoromethanesulfonate were slowly added. Once the filling was completed, it was maintained at -4 °C for 15 minutes. The temperature was increased to 10°C and 100 mL of water were added. It was stirred and decanted. The organic phase was vacuum distilled to obtain a residue.

### B) 6-fluoro-9β,11β-epoxy-17α,21-dihydroxy-16β-methylpregna-1,4-diene-3,20-dione 21-acetate

The residue obtained in the previous distillation was mixed with 100 mL of acetonitrile and was cooled at 0°C under inert atmosphere. Then, 4.69 g of N-fluoropyridinium tetrafluoroborate were slowly added. Once the filling was completed, the suspension was maintained for 1 hour and was then precipitated on 200 mL of water and 5 mL of 20% ammonium hydroxide. It was filtered and washed. 7g of a product were obtained which was analyzed by HPLC in the following conditions:
Detector: UV 254 nm
Flow rate: 1 mL/min
Eluent: acetonitrile 40: water 60
Column : Nova-Pak® C₁₈

A ratio of the following compounds was obtained:
- Isomer 6α of the titer, 78%
- Isomer 6β of the titer, 5%

### Reference Example 6

### A) 9β,11β-epoxy-3,17,21-trihydroxy-16α-methylpregna-1,3,5-triene-20-one 3-benzoate-21-acetate

20g of 9β,11β-epoxy-17α-dihydroxy-16α-methylpregna-1,4-diene-3,20-dione 21-acetate, 28 mL of pyridine and 0.08 g of hydroquinone were mixed under inert atmosphere at 20 °C. The mixture was heated to 70°C and 8.4 mL of benzoyl chloride were then added. It was maintained for 3 hours and the temperature was again reduced to 40°C. 20 mL of methanol were added and the mixture was cooled at 20°C.

The reaction mixture was precipitated on a solution of 400 mL of water and 28 mL of 30% hydrochloric acid cooled at 0°C. 150 mL of methanol were added and the solution was maintained at 0°C for 1 hour. It was filtered and washed. 21g of solid were obtained.

### B) 6-fluoro-9β,11β-epoxy-17α,21-dihydroxy-16α-methylpregna-1,4-diene-3,20-dione 21-acetate

The previously obtained solid and 200 mL of acetonitrile were mixed under inert atmosphere and 2 mL of water were added. The suspension was cooled at 0°C, and then, 18 g of N-fluoro-N-chloromethyl-triethylenediamine bis tetrafluoroborate were slowly added. When the filling was completed, the suspension was maintained at 0°C for 1 hour and, then a solution of 400 mL of water and 10 mL of 20% ammonia were added thereto. Subsequently, 0.4 g of sodium metabisulfite were added. It was filtered and washed. 13.2 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 84%
- Isomer 6β of the titer, 7%

### Reference Example 7

### A) 9β,11β-epoxy-3-t-butyl-dimethylsilyloxy-17α,21-dihydroxy-16β-methylpregna-1,3,5-triene-20-one 21-acetate

10 g of 9β,11β-epoxy-17α,21-dihydroxy-16β-methylpregna-1,4-diene-3,20-dione 21-acetate, 6.1 mL of diisopropylethylamine and 100 mL of dichloromethane were mixed at 20°C under inert atmosphere at -4°C. Then, 7.49 mL of t-butyldimethylsilyl trifluoromethanesulfonate were slowly added. Once the filling was completed, it was maintained at-4°C for 15 minutes. The temperature was increased to 10°C and 100 mL of water were added. It was stirred and decanted. The organic phase was vacuum distilled to obtain a residue.

### B) 6-fluoro-9β,11β-epoxy-17α,21-dihydroxy-16β-methylpregna-1,4-diene-3,20-dione 21-acetate

The residue obtained in the distillation was mixed with 180 mL of methanol, 20 mL of water and 1.1 mL of pyridine under inert atmosphere. The suspension was cooled at 0°C, and then, 9.4 g of N-fluoro-N-chloromethyl-triethylenediamine bis tetrafluoroborate were added. Once the filling was completed, it was maintained at 0°C for 1 hour and was filtered. The wet cake was suspended in 200 mL of water, and a sufficient quantity of 20% ammonium hydroxide was added to adjust the pH to 8. 2 g of sodium metabisulfite were added, and it was readjusted to pH 8. It was filtered and washed. 5 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds is obtained:
- Isomer 6α of the titer, 80%
- Isomer 6β of the titer, 15%

### Examples of the Invention

### Example 1

### 9β,11β-epoxy-3-t-butyl-dimethylsilyloxy-17α,21-dihydroxy-16β-methylpregna-1,3,5-triene-20-one 21-acetate

100g of 9β,11β-epoxy-17α,21-dihydroxy-16β-methylpregna-1,4-diene-3,20-dione 21-acetate, 61 mL of diisopropylethylamine and 1000 mL of dichloromethane were mixed at 20°C and cooled at -4°C. Following this, 74.9 mL of t-butyldimethylsilyl trifluoromethanesulfonate were slowly added. Once the filling was completed, it was maintained at-4°C for 15 minutes.

The temperature was increased to 10°C and 1000 mL of water were added. It was stirred and decanted. The organic phase was vacuum distilled and was replaced with 200 ml of acetonitrile. The resulting mixture was maintained at 0°C for 1 hour and was filtered and washed to obtain 122 g of the expected product.
Yield: 96% molar
NMR (CDCl₃): 400 MHz, ppm: 0.1 (s): Si-CH₃; 0.7 (s): 16-CH₃; 0.77 (s): 18-CH₃; 0.84 (s): Si-C-CH₃; 1.1 (s): 18-CH₃; 2.1 (s): CO-CH₃; 2.9 (s): H at position 11; 4.6, 5.05 (d, d): 2H at position 21; 5.2 (d): H at position 4; 5.32 (d): H at position 1; 5.4 (d): H at position 6; 5.54 (dd): H at position 2.

### Example 2

### 6-fluoro-9β,11β-epoxy-17α,21-dihydroxy-16β-methylpregna-1,4-diene-3,20-dione 21-acetate

5g of 9β,11β-epoxy-3-t-butyl-dimethylsilyloxy-17α,21-dihydroxy-16β-methylpregna-1,3,5-triene-20-one 21-acetate, were mixed together with 0.15 ml of pyridine and 50 mL of dichloromethane at 20°C under inert atmosphere. Maintaining temperature between 15 and 20°C, 3.14 g of N-fluorobenzenesulfonimide were slowly added to said mixture.

Once the filling was completed, the suspension was maintained at 15-20°C for 2 hours. Once this period elapsed, 100 ml of dichloromethane and 100 ml of water were added to the mixture, it was stirred for 30 minutes at 25-30 °C and was left to settle for 60 minutes. The solvent was vacuum removed from the lower organic phase until dryness, and the resulting residue crystallized into methanol. 3.5 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 92%
- Isomer 6β of the titer, 1%
NMR (CDCl₃): 400 MHz, ppm: 0.7 (s): 16-CH₃; 0.77 (s): 18-CH₃; 1.1 (s): 18-CH₃; 2.1 (s): CO-CH₃; 2.9 (s): H at position 11; 4.6, 5.05 (d, d): 2H at position 21; 5.4 (dddd): H at position 6β; 6.2 (dd): H at position 2; 6.3 (t): H at position 4; 6.6 (dd): H at position 1.

### Example 3

### 9β,11β-epoxy-3-t-butyl-dimethylsilyloxy-17α,21-dihydroxy-16α-methylpregna-1,3,5-triene-20-one 21-acetate

2 g of 9β,11β-epoxy-17α,21-dihydroxy-16α-methylpregna-1,4-diene-3,20-dione 21-acetate, 1.2 mL of diisopropylethylamine and 20 mL of dichloromethane were mixed at 20°C and cooled at -4°C. Then, 1.5 mL of t-butyldimethylsilyl trifluoromethanesulfonate were slowly added. Once the filling was completed, it was maintained at-4°C for 15 minutes. The temperature was increased to 10°C and 20 mL of water were added. It was stirred and decanted. The organic phase was vacuum distilled until obtaining an oil.

### B) 6-fluoro-9β,11β-epoxy-17α,21-dihydroxy-16α-methylpregna-1,4-diene-3,20-dione 21-acetate

The oil obtained in the distillation, together with 0.4 ml of pyridine and 20 mL of dichloromethane were mixed at 20°C under inert atmosphere. Maintaining the temperature between 15°C and 20°C, 1.6 g of N-fluorobenzenesulfonimide were slowly added to said mixture. Once the filling was completed, the suspension was maintained for 2 hours at 15-20°C. Once the reaction was finished, 50 mL of dichloromethane and 70 mL of water were added. The mixture was stirred for 15 minutes and the phases were left to be decanted for 30 minutes. The lower organic phase was separated, and the solvent was vacuum distilled until dryness. 1.5 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 92%
- Isomer 6β of the titer, 4%

### Example 4

### A) 3-t-butyl-dimethylsilyloxy-17α,21-dihydroxy-16α-methylpregna-1,3,5,9(11)-tetraene-20-one 21-acetate

10 g of 17α,21-dihydroxy-16α-methylpregna-1,4,9(11)-triene-3,20-dione 21-acetate, 6.1 mL of diisopropylethylamine and 100 mL of dichloromethane were mixed at 20°C under inert atmosphere at -4°C. Then, 7.5 mL of t-butyldimethylsilyl trifluoromethanesulfonate were slowly added. Once the filling was completed, it was maintained at -4°C for 15 minutes. The temperature was increased to 10°C and 100 mL of water were added. It was stirred and decanted. The organic phase was vacuum distilled.

### B) 6-fluoro-17α,21-dihydroxy-16α-methylpregna-1,4,9(11)-triene-3,20-dione 21-acetate

The residue obtained in the distillation of the previous step, together with 0.98 mL of pyridine and 10 mL of 1,2 dichloroethane were mixed at 20°C under inert atmosphere. The mixture was cooled at -2°C, and then, 8.3 g of N-fluorobenzenesulfonimide were slowly added. Once the filling was completed, the suspension was maintained at 0°C for 2 hours.

The suspension was vacuum distilled and was replaced with 30 mL of isopropanol, it was stirred for 1 hour at 0°C and filtered and washed. 6.2 g of a product were obtained which were analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 76%
- Isomer 6β of the titer, 1%

### Example 5

### A) 9β,11β-epoxy-3-triisopropylsilyloxy-17α,21-dihydroxy-16α-methylpregna-1,3,5-triene-20-one 21-acetate

10g of 9β, 11β-epoxy-17α,21-dihydroxy-16α-methylpregna-1,4-diene-3,20-dione 21-acetate, 6.1 mL of diisopropylethylamine and 100 mL of dichloromethane were mixed at 20°C under inert atmosphere at -4°C. Then, 7.5 ml of triisopropylsilyl trifluoromethanesulfonate were slowly added. Once the filling was completed, it was maintained at -4°C for 15 minutes. The temperature was increased to 10°C and 100 mL of water were added. It was stirred and decanted. The organic phase was vacuum distilled until obtaining an oil.

### B) 6-fluoro-9β,11β-epoxy-17α,21-dihydroxy-16α-methylpregna-1,4-diene-3,20-dione 21-acetate

The residue obtained in the distillation, together with 9.8 mL of pyridine and 100 mL of dichloromethane were mixed at 20°C under inert atmosphere. The mixture was stirred at 15-20°C and, then, 8 g of N- fluorobenzenesulfonimide were slowly added. Once the reaction was finished, 150 mL of dichloromethane and 50 mL of water were added. The mixture was stirred for 15 minutes and the phases were left to decant for 30 minutes. The lower organic phase was separated and the solvents were vacuum distilled until dryness. The resulting residue was recrystallized in methanol. 8.3 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 96%
- Isomer 6β of the titer, 1%

### Example 6

### A) 9B,11B-epoxy-3-t-butyldimethylsilyloxy-17α,21-dihydroxy-16α-methylpregna-1,3,5-triene-20-one 21-pivalate

10g of 9β,11β-epoxy-17α,21-dihydroxy-16α-methylpregna-1,4-diene-3,20-dione 21-pivalate, 5.5 mL of diisopropylethylamine and 100 mL of dichloromethane were mixed at 20°C under inert atmosphere and were cooled at -4°C. Then, 6.8 mL of t-butyldimethylsilyl trifluoromethanesulfonate were slowly added. Once the filling was completed, it was maintained at -4°C for 15 minutes. The temperature was increased to 10°C and 100 mL of water were added. It was stirred and decanted. The organic phase was vacuum distilled and used in the subsequent step.

### B) 6-fluoro-9β,11β-epoxy-17α,21-dihydroxy-16α-methylpregna-1,4-diene-3,20-dione 21-pivalate

The residue obtained in the distillation, together with 8.8 mL of pyridine and 100 mL of dichloromethane were mixed at 20°C under inert atmosphere. The mixture was cooled to 2°C, and then, 7.3 g of N-fluorobenzenesulfonimide were slowly added. Once the filling was completed, the suspension was maintained for 2 hours at 15-20°C. The solution was vacuum distilled, obtaining a reaction crude which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 97%
- Isomer 6β of the titer, 2%

### Example 7

### A) 9β,11β-epoxy-3-t-butyldimethylsilyloxy-17α,21-dihydroxy-16a-methylpregna-1,3,5-triene-20-one 21-propionate

10g of 9β,11β-epoxy-17α,21-dihydroxy-16α-methylpregna-1,4-diene-3,20-dione 21-pivalate, 5.9 ml of diisopropylethylamine and 100 mL of dichloromethane were mixed at 20°C under inert atmosphere and were cooled to -4°C. Then, 7.3 mL of t-butyldimethylsilyl trifluoromethanesulfonate were slowly added. Once the filling was completed, it was maintained at -4°C for 15 minutes. The temperature was increased to 10°C and 100 mL of water were added. It was stirred and decanted. The organic phase was vacuum distilled and used in the subsequent step.

### B) 6-fluoro-9β,11β-epoxy-17α,21-dihydroxy-16α-methylpregna-1,4-diene-3,20-dione 21-propionate

The residue obtained in the distillation, together with 9.4 mL of pyridine and 100 mL of 1,2 dichloroethane were mixed at 20°C under inert atmosphere. The mixture was cooled to-2°C, and then, 7.7 g of N-fluorobenzenesulfonimide were slowly added. Once the filling was completed, the suspension was maintained for 2 hours at 0°C. 5.9 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 95%
- Isomer 6β of the titer, 2%

### Example 8

### A) 9β,11β-epoxy-3,21-di(t-butyldimethylsilyloxy)-17α-hydroxy-16α-methylpregna-1,3,5-triene-20-one

10 g of 9,11β-epoxy-16α-methyl-Δ1,4-pregnadiene-17α,21-ol-3,20-one, 13.5 ml of diisopropylethylamine and 100 mL of dichloromethane were mixed at 20°C under inert atmosphere and cooled to -4°C. Then, 16.6 mL of t-butyldimethylsilyl trifluoromethanesulfonate were slowly added. Once the filling was completed, it was maintained at -4°C for 15 minutes. The temperature was increased to 10°C and 100 mL of water were added. It was stirred and decanted. The organic phase was vacuum distilled and used in the subsequent step.

### B) 6-fluoro-9β,11β-epoxy-17α,21-t-butyldimethylsilyloxy-16α-methylpregna-1,4-diene-3,20-dione

The residue obtained in the distillation, together with 1.9 mL of pyridine and 100 mL of dichloromethane were mixed at 20°C under inert atmosphere. The mixture was cooled to -2°C, and then, 8.9 g of N-fluorobenzenesulfonimide were slowly added. When the filling was completed, the suspension was maintained for 2 hours at 0°C. It was vacuum distilled until obtaining a residue analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 95%
- Isomer 6β of the titer, 2%

### Example 9

### A) 9β,11β-epoxy-3-t-butyldimethylsilyloxy-17α,21-dihydroxy-16α-methylpregna-1,3,5-triene-20-one 21-acetate

10 g of 9β,11β-epoxy-17α,21-dihydroxy-16α-methylpregna-1,4-diene-3,20-dione 21-acetate, 6.1 mL of diisopropylethylamine and 100 mL of dichloromethane were mixed at 20°C and cooled to -4°C. Then, 7.49 mL of t-butyldimethylsilyl trifluoromethanesulfonate were slowly added. Once the filling was completed, it was maintained at-4°C for 15 minutes. The temperature was increased to 10°C and 100 mL of water were added. It was stirred and decanted. The organic phase was vacuum distilled until obtaining an oil.

### B) 6-fluoro-9β,11β-epoxy-17α,21-dihydroxy-16α-methylpregna-1,4-diene-3,20-dione 21-acetate

The oil obtained in the distillation, together with 1.3 g of imidazole and 100 mL of dichloromethane were mixed at 20°C under inert atmosphere. Maintaining the temperature between 15 and 20°C, 8 g of N-fluorobenzenesulfonimide were slowly added to the mixture. Once the filling was completed, the suspension was maintained for 2 hours at 15-20°C. Once the reaction was completed, 150 mL of dichloromethane and 50 mL of water were added. The mixture was stirred for 15 minutes and the phases were left to decant for 30 minutes. The lower organic phase was separated by vacuum distillation of the solvents to dryness. The resultant residue was recrystallized into methanol. 8.2 g of a product were obtained which was analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 90%
- Isomer 6β of the titer, 2,5%

### Example 10

### A) 9β,11β-epoxy-3-t-butyldimethylsilyloxy-17α,21-dihydroxy-16α-methylpregna-1,3,5-triene-20-one 21-mesylate

10 g of 9β,11β-epoxy-17α,21-dihydroxy-16β-methylpregna-1,4-diene-3,20-dione 21-mesylate, 5 ml of diisopropylethylamine and 100 mL of dichloromethane were mixed at 20°C under inert atmosphere and cooled to -4°C; Then, 6 mL of t-butyldimethylsilyl trifluoromethanesulfonate were slowly added. Once the filling was completed, it was maintained at -4°C for 15 minutes. The temperature was increased to 10°C and 100 mL of water were added. It was stirred and decanted. The organic phase was vacuum distilled and was used in the subsequent step.

### B) 6-fluoro-9β,11β-epoxy-17α,21-dihydroxy-16α-methylpregna-1,4-diene-3,20-dione 21-mesylate

The residue obtained in the distillation, together with 0.8 mL of pyridine and 100 mL of 1,2 dichloroethane were mixed at 20 °C under inert atmosphere. The mixture was cooled at -2°C, and then, 6.6 g of N-fluorobenzenesulfonimide were slowly added. Once the filling was completed, the suspension was maintained for 2 hours at 0°C. It was vacuum distilled until obtaining a residue analyzed by HPLC in the following conditions:
Detector: UV 254 nm
Flow rate: 1 mL/min
Eluent: acetonitrile 40: water 60
Column: Nova-Pak® C₁₈

A ratio of the following compounds was obtained:
- Isomer 6α of the titer, 78%
- Isomer 6β of the titer, 6%

## Claims

1. A process for the production of 6α-fluorpregnanes, of general formula (I): 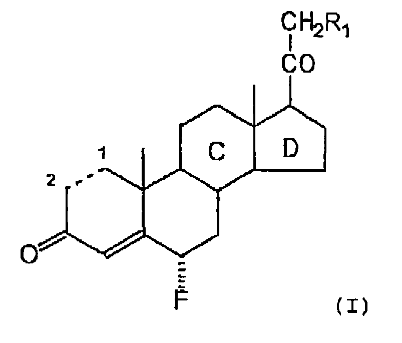 where
the dotted line between positions 1 and 2 represents a single or double bond;
R₁ is OH, OCOR₂, X, SO₃R₃ or an (R₇)(R₈)(R₉)SiO- group, where X is halogen, R₂ and R₃ are C₁₋₆ alkyl or phenyl optionally substituted by C₁₋₄ alkyl, and R₇, R₈ and R₉, equal or different, are C₁₋₆ alkyl or phenyl optionally substituted by C₁₋₄ alkyl;
the C ring of the steroid is: 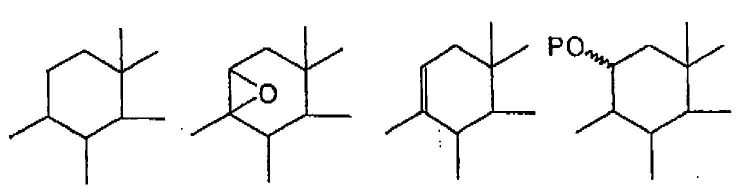
where
P is a protector group of the hydroxyl group; and
the D ring of the steroid is: 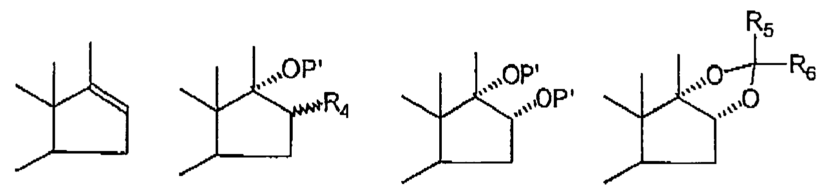
where
R₄ is H or CH₃ (α or β configuration);
R₅ and R₆, equal or different, are C₁₋₄ alkyl; and
each P', independently, is H, a protector group of the hydroxyl or an (R₇)(R₈)(R₉)Si- group, where R₇, R₈ and R₉ have the previously mentioned meaning;
comprising reacting a 3-(trisubstituted)silyloxy-pregna-3,5-diene of general formula (IV): 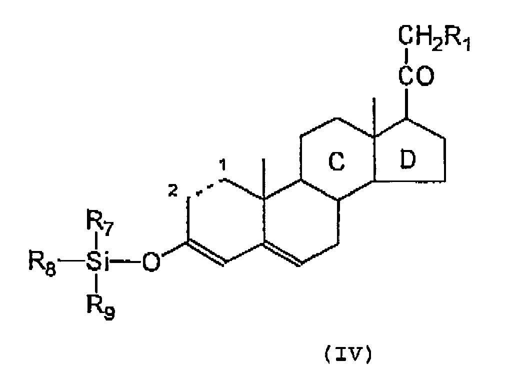 where
the dotted line between positions 1 and 2, R₁, R₇, R₈ and R₉, and the C and D rings of the steroid, have the previously mentioned meaning,
with a fluorinating agent selected among:
(i) an N-fluorosulfonimide of general formula (V)
R₁₀-O₂S-NF-SO₂-R₁₁ (V)
where
R₁₀ and R₁₁, equal or different, are C₁₋₄ alkyl with one or more hydrogen atoms optionally substituted by halogen, or phenyl optionally substituted by C₁₋₄ alkyl;
(ii) an N-fluorosulfonimide of general formula (VI) 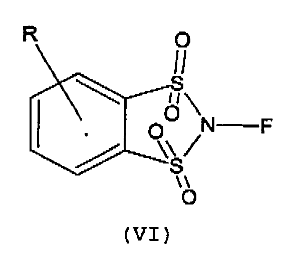 where
R is a C₁₋₆ alkyl radical; and
(iii) an N-fluorosulfonamide of general formula (VII) 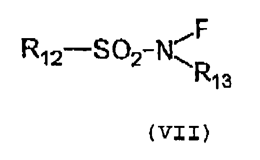 where
R₁₂ is phenyl optionally substituted by C₁₋₄ alkyl; and
R₁₃ is H, C₁₋₆ alkyl or phenyl optionally substituted by C₁₋₄ alkyl.

2. A process according to claim 1, for the production of a compound of formula (I) wherein the dotted line between positions 1 and 2 represents a double bond.

3. A process according to claim 1, for the production of a compound of formula (I) wherein R₁ is hydroxyl, acetate, pivalate, propionate, mesylate or chlorine.

4. A process according to claim 1, for the production of a compound of formula (I) presenting a 9β,11β-epoxy group in the C ring, or a double bond between positions 9 and 11 of the C ring.

5. A process according to claim 1, for the production of a compound of formula (I) wherein R₄ is H, αCH₃ or βCH₃.

6. A process according to claim 1, for the production of a compound of formula (I) containing an αOH group at position 17.

7. A process according to claim 1, for the production of a compound of formula (I) wherein R₅ and R₆ are, simultaneously, methyl.

8. A process according to claim 1, for the production of a compound of formula (I) wherein the dotted line between positions 1 and 2 represents a double bond, R₁ is hydroxyl, acetate, pivalate, propionate, mesylate or chlorine, has a 9β,11β-epoxy group in the C ring, R₄ is H, αCH₃ or βCH₃, and has an αOH group at position 17.

9. A process according to claim 1, for the production of a compound of formula (I) wherein the dotted line between positions 1 and 2 represents a double bond, R₁ is hydroxyl, acetate, pivalate, propionate, mesylate or chlorine, has a double bond between positions 9 and 11, R₄ is H, αCH₃ or βCH₃, and has an αOH group at position 17.

10. A process according to claim 1, wherein the reaction between the compound of formula (IV) and the fluorinating agent selected among the compounds of formula (V), (VI) and (VII) is carried out in an organic solvent selected among a halogenated organic solvent, an aromatic hydrocarbon, an ether and acetonitrile.

11. A process according to claim 10, wherein said halogenated organic solvent is methylene chloride, 1,2-dichloroethane or chloroform.

12. A process according to claim 1, wherein the reaction between the compound of formula (IV) and the fluorinating agent selected among the compounds of formula (V), (VI) and (VII) is carried out in the presence of a nitrogenated organic base.

13. A process according to claim 12, wherein the nitrogenated organic base is triazole, aminotriazole, imidazole or pyridine.

14. A process according to claim 1, wherein the reaction between the compound of formula (IV) and the fluorinating agent selected among the compounds of formula (V), (VI) and (VII) is carried out at a temperature comprised between -40°C and +20°C, preferably between -10°C and 0°C.

15. A compound of general formula (IV): 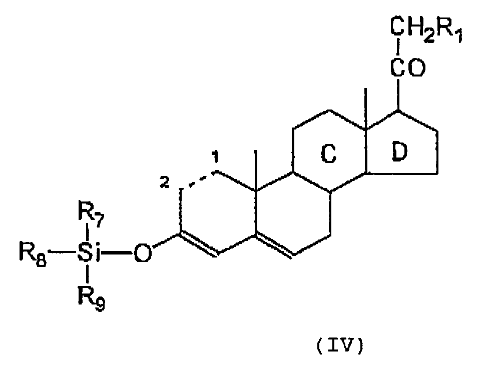 where
the dotted line between positions 1 and 2 represents a single or double bond;
R₁ is OH, OCOR₂, X, SO₃R₃ or an (R₇)(R₈)(R₉)SiO- group, where X is halogen, R₂ and R₃ are C₁₋₆ alkyl or phenyl optionally substituted by C₁₋₄ alkyl, and R₇, R₈ and R₉, equal or different, are C₁₋₆ alkyl or phenyl optionally substituted by C₁₋₄ alkyl;
the C ring of the steroid is: 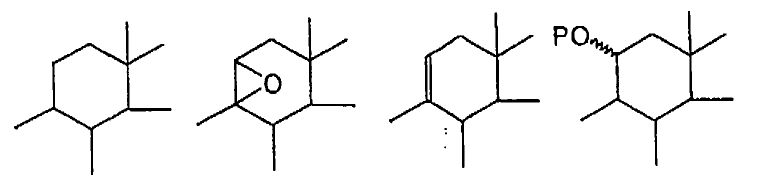
where
P is a protector group of the hydroxyl group; and
the D ring of the steroid is: 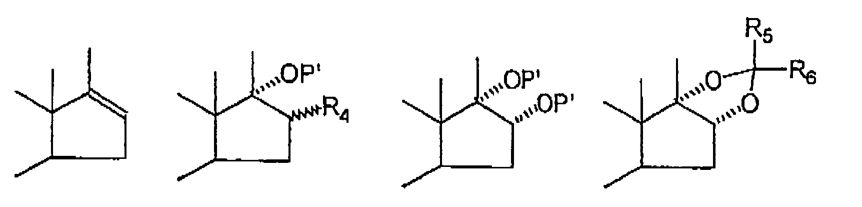
where
R₄ is H or CH₃ (α or β configuration);
R₅ and R₆, equal or different, are C₁₋₄ alkyl; and
each P', independently, is H, a protector group of the hydroxyl or an (R₇)(R₈)(R₉)Si- group, where R₇, R₈ and R₉ have the previously mentioned meaning.

16. A compound according to claim 15, wherein the dotted line between positions 1 and 2 represents a double bond.

17. A compound according to claim 15, wherein R₁ is acetate, pivalate, propionate or mesylate.

18. A compound according to claim 15, having a 9β,11β-epoxy group in the C ring or a double bond between positions 9 and 11 of the C ring.

19. A compound according to claim 15, wherein R₄ is H, αCH₃ or βCH₃.

20. A compound according to claim 15, containing an αOH group at position 17.

21. A compound according to claim 15, wherein R₅ and R₆ are simultaneously methyl.

22. A compound according to claim 15, wherein two groups selected among R₇, R₈ and R₉ are simultaneously methyl and the other one is t-butyl, or wherein R₇, R₈ and R₉ are simultaneously isopropyl.

23. A compound according to claim 15, wherein the dotted line between positions 1 and 2 represents a double bond, R₁ is acetate, pivalate, propionate or mesylate, it has a 9β,11β-epoxy group in the C ring, R₄ is αCH₃ or βCH₃, it has an αOH group at position 17, two groups selected among R₇, R₈ and R₉ are simultaneously methyl and the other one is t-butyl, or R₇, R₈ and R₉ are simultaneously isopropyl.

24. A compound according to claim 15, wherein the dotted line between positions 1 and 2 represents a double bond, R₁ is acetate, pivalate, propionate or mesylate, it has a double bond between positions 9 and 11, R₄ is αCH₃ or βCH₃, it has an αOH group at position 17, two groups selected among R₇, R₈ and R₉ are simultaneously methyl and the other one is t-butyl, or R₇, R₈ and R₉ are simultaneously isopropyl.

25. A compound according to claim 15, containing an (R₇)(R₈)(R₉)SiO- group at position 16 and/or 21.

26. A process for obtaining a compound of formula (IV) according to any of claims 15 to 25, comprising reacting a pregnane derivative of general formula (II): 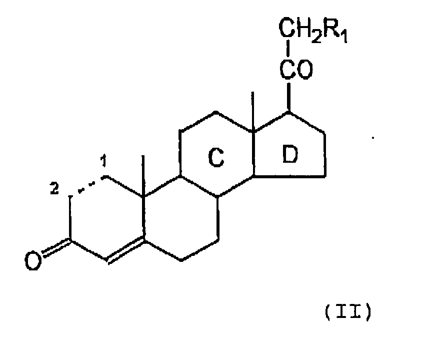 where
the dotted line between positions 1 and 2 represents a single or double bond;
R₁ is OH, OCOR₂, X, SO₃R₃, or an (R₇)(R₈)(R₉)SiO- group, where X is halogen, R₂ and R₃ are C₁₋₆ alkyl or phenyl optionally substituted by C₁₋₄ alkyl, and R₇, R₈ and R₉, equal or different, are C₁₋₆ alkyl or phenyl optionally substituted by C₁₋₄ alkyl;
the C ring of the steroid is: 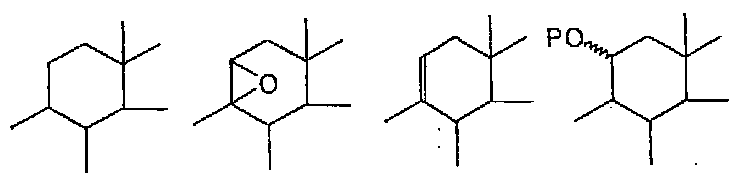
where
P is a protector group of the hydroxyl group; and
the D ring of the steroid is: 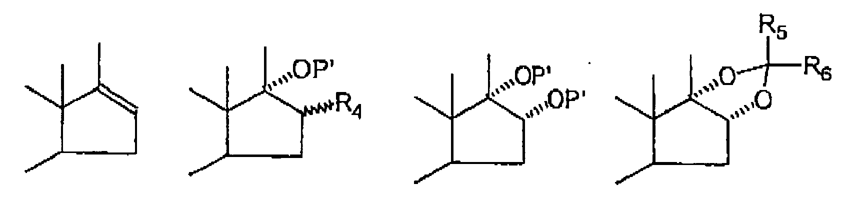
where
R₄ is H or CH₃ (α or β configuration);
R₅ and R₆, equal or different, are C₁₋₄ alkyl; and
each P', independently, is H, a protector group of the hydroxyl or an (R₇) (R₈) (R₉) Si- group, where R₇, R₈ and R₉ have the previously mentioned meaning;
with a (trisubstituted)silyl trifluoromethanesulfonate of general formula (III): 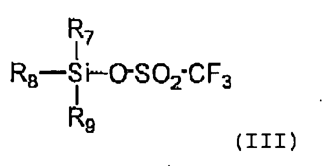 where
R₇, R₈ and R₉ have the previously mentioned meaning.

27. A process according to claim 26, wherein said compound of formula (III) is t-butyldimethylsilyl trifluoromethanesulfonate or triisopropylsilyl trifluoromethanesulfonate.

28. A process according to claim 26, wherein the reaction between the compound of formula (II) and the compound of formula (III) is carried out in an organic solvent selected among a halogenated organic solvent, an ether and acetonitrile.

29. A process according to claim 28, wherein said halogenated solvent is dichloromethane or 1,2-dichloroethane.

30. A process according to claim 26, wherein the reaction between the compound of formula (II) and the compound of formula (III) is carried out in the presence of a nitrogenated organic base.

31. A process according to claim 30, wherein said nitrogenated organic base is diisopropylethylamine, triethylamine, lutidine or collidine.

32. A process according to claim 26, wherein the reaction between the compound of formula (II) and the compound of formula (III) is carried out at a temperature comprised between -20°C and 25°C, preferably between -10°C and 0°C.

33. A process according to claim 26, wherein the reaction between the compound of formula (II) and the compound of formula (III) is carried out at a compound (III):compound (II) molar ratio equal to or greater than 2 to obtain the disilylated derivative of the compound of formula (IV), or equal to or greater than 3 to obtain the trisilylated derivative of the compound of formula (IV).

34. A process according to claim 26, wherein the compound of formula (II) contains an (R₇)(R₈)(R₉)SiO- group, where R₇, R₈ and R₉, equal or different, are C₁₋₆ alkyl or phenyl optionally substituted by C₁₋₄ alkyl at position 16 and/or 21, and the reaction between said compound of formula (II) and the compound of formula (III) is carried out at a suitable molar ratio to obtain the disilylated derivative or the trisilylated derivative of the compound of formula (IV).

35. A process according to claim 1 for the production of 6α-fluorpregnane (I) : 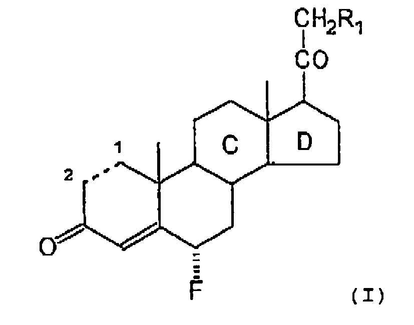 where
the dotted line between positions 1 and 2 represents a single or double bond;
R₁ is OH, OCOR₂, X, SO₃R₃, or an (R₇)(R₈)(R₉)SiO- group, where X is halogen, R₂ and R₃ are C₁₋₆ alkyl or phenyl optionally substituted by C₁₋₄ alkyl, and R₇, R₈ and R₉, equal or different, are C₁₋₆ alkyl or phenyl optionally substituted by C₁₋₄ alkyl;
the C ring of the steroid is: 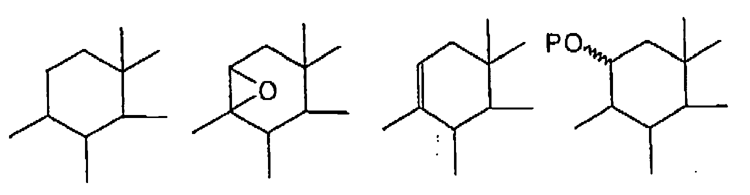
where
P is a protector group of the hydroxyl group; and
the D ring of the steroid is: 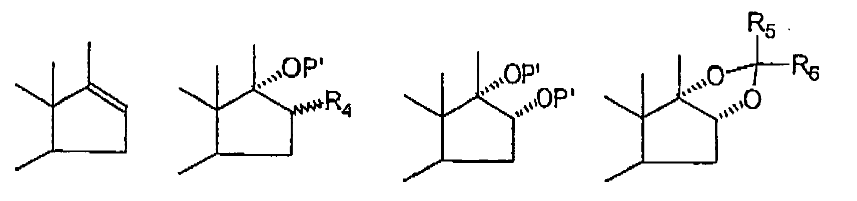
where
R₄ is H or CH₃ (α or β configuration);
R₅ and R₆, equal or different, are C₁₋₄ alkyl; and
each P', independently, is H, a protector group of the hydroxyl or an (R₇)(R₈)(R₉)Si-] group, where R₇, R₈ and R₉ have the previously mentioned meaning;
the process further comprising the production of the compound of formula (IV) by reacting a pregnane derivative of general formula (II) 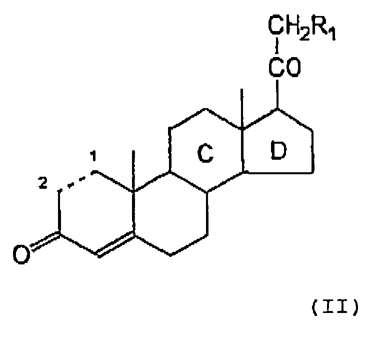 where
the dotted line between positions 1 and 2, R₁ and the C and D rings have the previously mentioned meanings,
with a (trisubstituted)silyl trifluoromethanesulfonate of general formula (III): 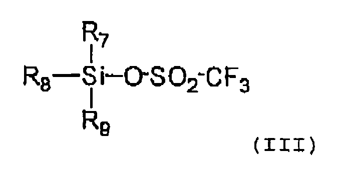 where
R₇, R₈ and R₉ have the previously mentioned meanings,
to obtain a compound of formula (IV) 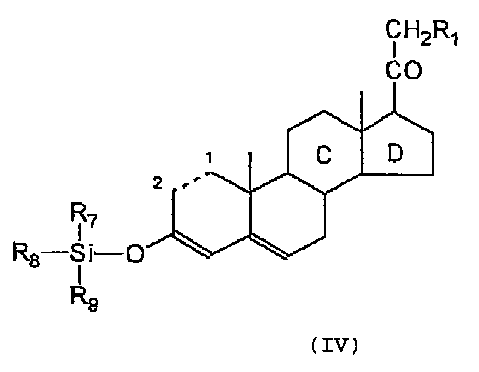 where
the dotted line between positions 1 and 2, R₁, R₇, R₈, R₉, and the C and D rings have the previously mentioned meanings.

36. A process according to claim 35, comprising the isolation of the compound of formula (IV) formed by reaction of the compound of formula (II) with the compound of formula (III) prior to its reaction with the fluorinating agent.

37. A process according to claim 35, wherein the reaction of the compound of formula (IV) with the compound of formula (V), (VI) or (VII) takes place without the isolation of the compound of formula (IV) formed by reaction of the compound of formula (II) with the compound of formula (III).

38. A process according to claim 37, comprising the removal of the water soluble contaminants generated after the reaction of the compound of formula (II) with the compound of formula (III) to form the compound of formula (IV) and prior to the reaction of the latter with the compound of formula (V), (VI) or (VII).

## Patentansprüche

1. Verfahren zur Herstellung von 6α-Fluorpregnanen der allgemeinen Formel (I): 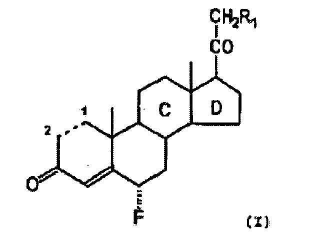 worin
die gepunktete Linie zwischen den Positionen 1 und 2 eine Einfach- oder Doppelbindung darstellt;
R₁ OH, OCOR₂, X, SO₃R₃ oder eine (R₇)(R₈)(R₉)SiO-Gruppe ist, worin X Halogen ist, R₂ und R₃ C₁₋₆Alkyl oder optional mit C₁₋₄Alkyl substituiertes Phenyl sind, und R₇, R₈ und R₉, gleich oder verschieden, C₁₋₆Alkyl oder optional mit C₁₋₄Alkyl substituiertes Phenyl sind;
der Ring C des Steroids 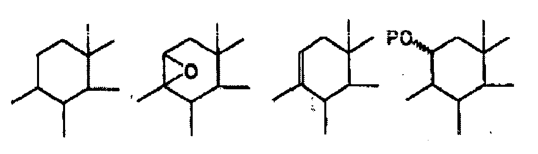
ist, worin
P eine Schutzgruppe der Hydroxylgruppe ist; und der Ring D des Steroids 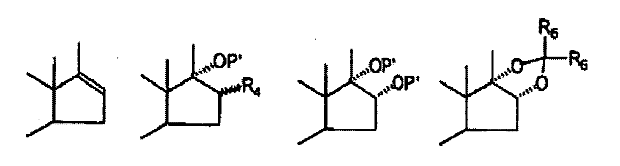
ist, worin
R₄ H oder CH₃ ist (α- oder β-Konfiguration);
R₅ und R₆, gleich oder verschieden, C₁₋₄Alkyl sind; und
jedes P', unabhängig, H ist, eine Hydroxylschutzgruppe oder eine (R₇)(R₈) (R₉)Si-Gruppe, worin R₇, R₈ und R₉ die zuvor genannte Bedeutung besitzen;
umfassend das Umsetzen eines 3-(trisubstituierten)Siloxy-pregna-3,5-diens der allgemeinen Formel (IV): 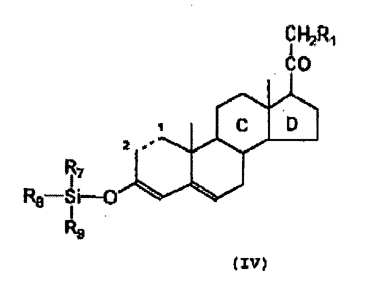
worin
die gepunktete Linie zwischen den Positionen 1 und 2, R₁, R₇, R₈ und R₉, und
die Ringe C und D des Steroids die zuvor genannte Bedeutung besitzen,
mit einem Fluorierungsmittel, ausgewählt aus:
(i) einem N-Fluorsulfonimid der allgemeinen Formel (V)
R₁₀-O₂S-NF-SO₂-R₁₁ (V)
worin
R₁₀ und R₁₁, gleich oder verschieden, C₁₋₄Alkyl sind, bei dem optional ein oder mehrere Wasserstoffatome durch Halogen substituiert sind, oder optional mit C₁₋₄Alkyl substituiertes Phenyl;
(ii) einem N-Fluorsulfonimid der allgemeinen Formel (VI) 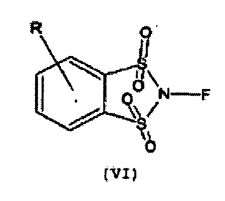 worin
R ein C₁₋₆Alkylradikal ist; und
(iii) einem N-Fluorsulfonamid der allgemeinen Formel (VII) 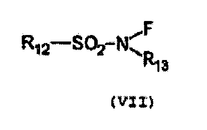 worin
R₁₂ optional mit C₁₋₄Alkyl substituiertes Phenyl ist; und
R₁₃ H, C₁₋₆Alkyl oder optional mit C₁₋₄Alkyl substituiertes Phenyl ist.

2. Verfahren gemäß Anspruch 1 zur Herstellung einer Verbindung der Formel (I), worin die gepunktete Linie zwischen den Positionen 1 und 2 eine Doppelbindung darstellt.

3. Verfahren gemäß Anspruch 1 zur Herstellung einer Verbindung der Formel (I), worin R₁ Hydroxyl ist, Acetat, Pivalat, Propionat, Mesylat oder Chlor.

4. Verfahren gemäß Anspruch 1 zur Herstellung einer Verbindung der Formel (I) mit einer 9β,11β-Epoxygruppe in Ring C oder einer Doppelbindung zwischen den Positionen 9 und 11 des Rings C.

5. Verfahren gemäß Anspruch 1 zur Herstellung einer Verbindung der Formel (I), worin R₄H, αCH3 oder βCH₃ ist.

6. Verfahren gemäß Anspruch 1 zur Herstellung einer Verbindung der Formel (I), enthaltend eine αOH-Gruppe an Position 17.

7. Verfahren gemäß Anspruch 1 zur Herstellung einer Verbindung der Formel (I), worin R₅ und R₆ gleichzeitig Methyl sind.

8. Verfahren gemäß Anspruch 1 zur Herstellung einer Verbindung der Formel (I), worin die gepunktete Linie zwischen den Positionen 1 und 2 eine Doppelbindung darstellt, R₁ Hydroxyl ist, Acetat, Pivalat, Propionat, Mesylat oder Chlor, sie eine 9β,11β-Epoxygruppe in Ring C besitzt, R₄ H, αCH3 oder βCH₃ ist, und eine αOH-Gruppe an Position 17 aufweist.

9. Verfahren gemäß Anspruch 1 zur Herstellung einer Verbindung der Formel (I), worin die gepunktete Linie zwischen den Positionen 1 und 2 eine Doppelbindung darstellt, R₁ Hydroxyl ist, Acetat, Pivalat, Propionat, Mesylat oder Chlor, sie eine Doppelbindung zwischen den Positionen 9 und 11 besitzt, R₄ H, αCH3 oder βCH₃ ist, und eine αOH-Gruppe an Position 17 aufweist.

10. Verfahren gemäß Anspruch 1, bei dem die Umsetzung zwischen der Verbindung der Formel (IV) und dem aus den Verbindungen der Formel (V), (VI) und (VII) ausgewählten Fluorierungsmittel in einem Lösungsmittel durchgeführt wird, das ausgewählt ist aus einem halogenierten organischen Lösungsmittel, einem aromatischen Kohlenwasserstoff, einem Ether und Acetonitril.

11. Verfahren gemäß Anspruch 10, bei dem das halogenierte organische Lösungsmittel Methylenchlorid, 1,2-Dichlorethan, oder Chloroform ist.

12. Verfahren gemäß Anspruch 1, bei dem die Umsetzung zwischen der Verbindung der Formel (IV) und dem aus den Verbindungen der Formel (V), (VI) und (VII) ausgewählten Fluorierungsmittel in Gegenwart einer stickstoffhaltigen organischen Base durchgeführt wird.

13. Verfahren gemäß Anspruch 12, bei dem die stickstofflialtige organische Base Triazol, Aminotriazol, Imidazol oder Pyridin ist.

14. Verfahren gemäß Anspruch 1, bei dem die Umsetzung zwischen der Verbindung der Formel (IV) und dem aus den Verbindungen der Formel (V), (VI) und (VII) ausgewählten Fluorierungsmittel bei einer Temperatur im Bereich zwischen -40°C und +20°C durchgeführt wird, bevorzugt zwischen -10°C und 0°C.

15. Verbindung der allgemeinen Formel (IV): 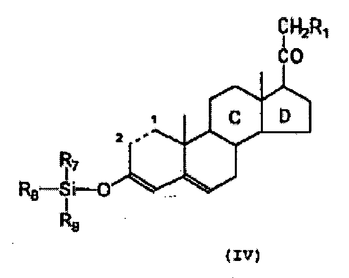 worin,
die gepunktete Linie zwischen den Positionen 1 und 2 eine Einfach- oder Doppelbindung darstellt;
R₁ OH, OCOR₂, X, SO₃R₃ oder eine (R₇)(R₈)(R₉)SiO-Gruppe ist, worin X Halogen ist, R₂ und R₃ C₁₋₆Alkyl oder optional mit C₁₋₄Alkyl substituiertes Phenyl sind, und R₇, R₈ und R₉, gleich oder verschieden, C₁₋₆Alkyl oder optional mit C₁₋₄Alkyl substituiertes Phenyl sind;
der Ring C des Steroids 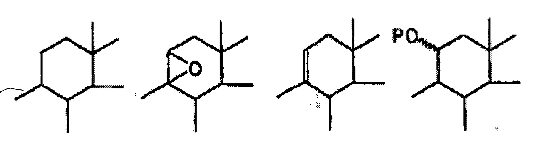
ist, worin
P eine Schutzgruppe der Hydroxylgruppe ist; und der Ring D des Steroids 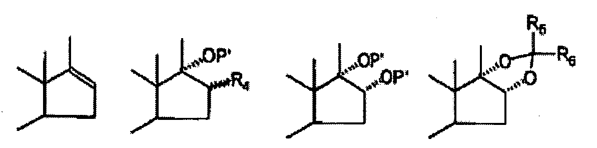
ist, worin
R₄ H oder CH₃ ist (α- oder β-Konfiguration);
R₅ und R₆, gleich oder verschieden, C₁₋₄Alkyl sind; und
jedes P', unabhängig, H ist, eine Hydroxylschutzgruppe oder eine (R₇)(R₈) (R₉)Si-Gruppe, worin R₇, R₈ und R₉ die zuvor genannte Bedeutung besitzen.

16. Verbindung gemäß Anspruch 15, worin die gepunktete Linie zwischen den Positionen 1 und 2 eine Doppelbindung darstellt.

17. Verbindung gemäß Anspruch 15, worin R₁ Acetat, Pivalat, Propionat oder Mesylat ist.

18. Verbindung gemäß Anspruch 15, mit einer 9β,11β-Epoxygruppe im Ring C oder mit einer Doppelbindung zwischen den Positionen 9 und 11 des Rings C.

19. Verbindung gemäß Anspruch 15, worin R₄ H, αCH3 oder βCH₃ ist.

20. Verbindung gemäß Anspruch 15, enthaltend eine αOH-Gruppe an Position 17.

21. Verbindung gemäß Anspruch 15, worin R₅ und R₆ gleichzeitig Methyl sind.

22. Verbindung gemäß Anspruch 15, worin zwei aus R₇, R₈ und R₉ ausgewählte Gruppen gleichzeitig Methyl sind und die andere t-Butyl ist, oder worin R₇, R₈ und R₉ gleichzeitig Isopropyl sind.

23. Verbindung gemäß Anspruch 15, worin die gepunktete Linie zwischen den Positionen 1 und 2 eine Doppelbindung darstellt, R₁ Acetat ist, Pivalat, Propionat oder Mesylat, sie eine 9β,11β-Epoxygruppe im Ring C besitzt, R₄ αCH3 oder βCH₃ ist, sie eine αOH-Gruppe an Position 17 besitzt, und zwei aus R₇, R₈ und R₉ ausgewählte Gruppen gleichzeitig Methyl sind und die andere t-Butyl ist, oder worin R₇, R₈ und R₉ gleichzeitig Isopropyl sind.

24. Verbindung gemäß Anspruch 15, worin die gepunktete Linie zwischen den Positionen 1 und 2 eine Doppelbindung darstellt, R₁ Acetat ist, Pivalat, Propionat oder Mesylat, sie eine Doppelbindung zwischen den Positionen 9 und 11 besitzt, R₄ αCH₃ oder βCH₃ ist, sie eine αOH-Gruppe an Position 17 besitzt, und zwei aus R₇, R₈ und R₉ ausgewählte Gruppen gleichzeitig Methyl sind und die andere t-Butyl ist, oder worin R₇, R₈ und R₉ gleichzeitig Isopropyl sind.

25. Verbindung gemäß Anspruch 15, enthaltend eine (R₇)(R₈)(R₉)SiO-Gruppe an Position 16 und/oder 21.

26. Verfahren zum Erhalt einer Verbindung der Formel (IV) nach einem der Ansprüche 15 bis 25, umfassend ein Umsetzen eines Pregnanderivats der allgemeinen Formel (II): 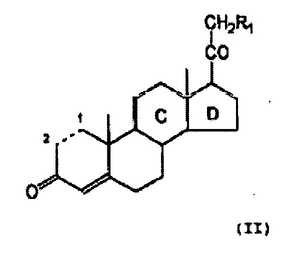 worin
die gepunktete Linie zwischen den Positionen 1 und 2 eine Einfach- oder Doppelbindung darstellt;
R₁ OH, OCOR₂, X, SO₃R₃ oder eine (R₇)(R₈)(R₉)SiO-Gruppe ist, worin X Halogen ist, R₂ und R₃ C₁₋₆Alkyl oder optional mit C₁₋₄Alkyl substituiertes Phenyl sind, und R₇, R₈ und R₉, gleich oder verschieden, C₁₋₆Alkyl oder optional mit C₁₋₄Alkyl substituiertes Phenyl sind;
der Ring C des Steroids 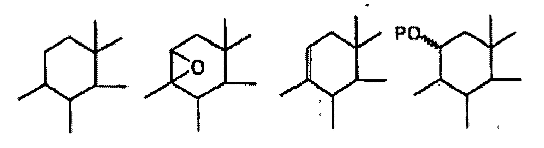
ist, worin
P eine Schutzgruppe der Hydroxylgruppe ist; und der Ring D des Steroids 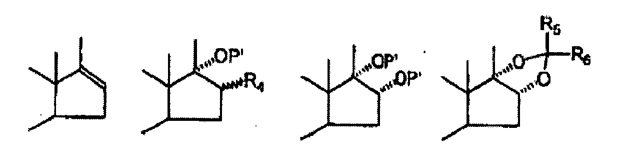
ist, worin
R₄ H oder CH₃ ist (α- oder β-Konfiguration);
R₅ und R₆, gleich oder verschieden, C₁₋₄Alkyl sind; und
jedes P', unabhängig, H ist, eine Hydroxylschutzgruppe oder eine (R₇)(R₈) (R₉)Si-Gruppe, worin R₇, R₈ und R₉ die zuvor genannte Bedeutung besitzen;
mit einem (trisubstituierten) Silyltrifluormethansulfonat der allgemeinen Formel (III): 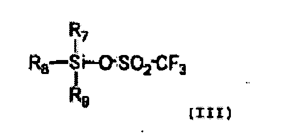
worin
R₇, R₈ und R₉ die zuvor genannte Bedeutung besitzen.

27. Verfahren gemäß Anspruch 26, bei dem die Verbindung der Formel (III) t-Butyldimethylsilyltrifluormethansulfonat oder Triisopropylsilyltrifluormethansulfonat ist.

28. Verfahren gemäß Anspruch 26, bei dem die Umsetzung zwischen der Verbindung der Formel (II) und der Verbindung der Formel (III) in einem organischen Lösungsmittel durchgeführt wird, das aus einem halogenierten organischen Lösungsmittel, einem Ether und Acetonitril ausgewählt ist.

29. Verfahren gemäß Anspruch 28, bei dem das halogenierte Lösungsmittel Dichlormethan oder 1,2-Dichlorethan ist.

30. Verfahren gemäß Anspruch 26, bei dem die Umsetzung zwischen der Verbindung der Formel (II) und der Verbindung der Formel (III) in Gegenwart einer stickstoffhaltigen organischen Base durchgeführt wird.

31. Verfahren gemäß Anspruch 30, bei dem die stickstoffhaltige Base Diisopropylethylamin, Triethylamin, Lutidin oder Collidin ist.

32. Verfahren gemäß Anspruch 26, bei dem die Umsetzung zwischen der Verbindung der Formel (II) und der Verbindung der Formel (III) bei einer Temperatur im Bereich zwischen -20°C und 25°C durchgeführt wird, bevorzugt zwischen -10°C und 0°C.

33. Verfahren gemäß Anspruch 26, bei dem die Umsetzung der Verbindung der Formel (II) mit der Verbindung der Formel (III) zum Erhalt des disylilierten Derivats der Verbindung der Formel (IV) bei einem Molverhältnis von Verbindung (III):Verbindung (II) von gleich oder größer als 2 durchgeführt wird, oder gleich oder größer als 3 zum Erhalt des trisylilierten Derivats der Verbindung der Formel (IV).

34. Verfahren gemäß Anspruch 26, bei dem die Verbindung der Formel (II) eine (R₇)(R₈)(R₉)SiO-Gruppe enthält, worin R₇, R₈ und R₉, gleich oder verschieden, C₁₋₆Alkyl oder optional mit C₁₋₄Alkyl substituiertes Phenyl an der Position 16 und/oder 21 sind, und die Umsetzung zwischen der Verbindung der Formel (II) und der Verbindung der Formel (III) bei einem geeigneten Molverhältnis zum Erhalt des disilylierten Derivats oder des trisilylierten Derivats der Verbindung der Formel (IV) durchgeführt wird.

35. Verfahren gemäß Anspruch 1 zur Herstellung von 6α-Fluorpregnan (I): 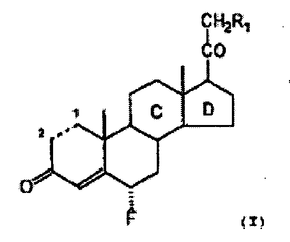 worin
die gepunktete Linie zwischen den Positionen 1 und 2 eine Einfach- oder Doppelbindung darstellt;
R₁ OH, OCOR₂, X, SO₃R₃ oder eine (R₇)(R₈)(R₉)SiO-Gruppe ist, worin X Halogen ist, R₂ und R₃ C₁₋₆Alkyl oder optional mit C₁₋₄Alkyl substituiertes Phenyl sind, und R₇, R₈ und R₉, gleich oder verschieden, C₁₋₆Alkyl oder optional mit C₁₋₄Alkyl substituiertes Phenyl sind;
der Ring C des Steroids 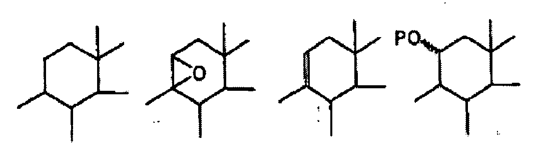
ist, worin
P eine Schutzgruppe der Hydroxylgruppe ist; und der Ring D des Steroids 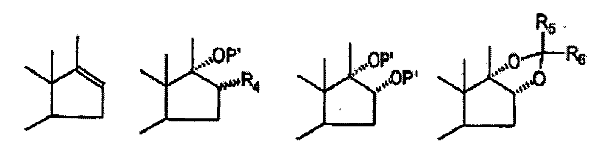
ist, worin
R₄ H oder CH₃ ist (α- oder β-Konfiguration);
R₅ und R₆, gleich oder verschieden, C₁₋₄Alkyl sind; und
jedes P', unabhängig, H ist, eine Hydroxylschutzgruppe oder eine (R₇)(R₈) (R₉)Si-Gruppe, worin R₇, R₈ und R₉ die zuvor genannte Bedeutung besitzen;
wobei das Verfahren ferner die Herstellung der Verbindung der Formel (IV) durch Umsetzen eines Pregnanderivats der allgemeinen Formel (II) 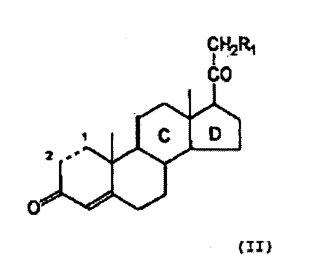
worin
die gepunktete Linie zwischen den Positionen 1 und 2, R₁ und die Ringe C und D die zuvor genannten Bedeutungen besitzen,
mit einem (trisubstituierten) Silyltrifluormethansulfonat der allgemeinen Formel (III) 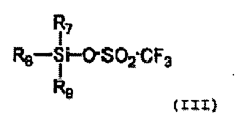
worin
R₇, R₈ und R₉ zuvor genannten Bedeutungen besitzen,
zum Erhalt einer Verbindung der Formel (IV) 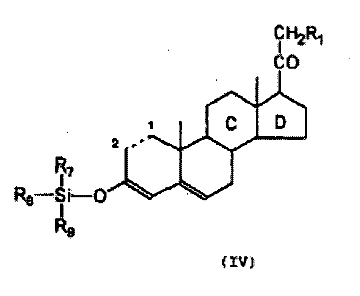
worin
die gepunktete Linie zwischen den Positionen 1 und 2, R₁, R₇, R₈ und R₉ und die Ringe C und D die zuvor genannten Bedeutungen besitzen.

36. Verfahren gemäß Anspruch 35, umfassend das Isolieren der Verbindung der durch Umsetzen der Verbindung der Formel (II) mit der Verbindung der Formel (III) gebildeten Verbindung der Formel (IV) vor ihrer Umsetzung mit dem Fluorierungsagens.

37. Verfahren gemäß Anspruch 35, bei dem die Umsetzung der Verbindung der Formel (IV) mit der Verbindung (V), (VI) oder (VII) ohne das Isolieren der Verbindung der durch Umsetzung der Verbindung der Formel (II) mit der Verbindung der Formel (III) gebildeten Verbindung der Formel (IV) stattfindet.

38. Verfahren gemäß Anspruch 37, umfassend das Entfernen der nach der Umsetzung der Verbindung der Formel (II) mit der Verbindung der Formel (III) zur Bildung der Verbindung der Formel (IV) erzeugten wasserlöslichen Verunreinigungen und vor Umsetzung der Verbindung der Formel (IV) mit der Verbindung der Formel (V), (VI) oder (VII).

## Revendications

1. Procédé pour la production de 6α-fluoroprégnanes, de formule générale (I) : 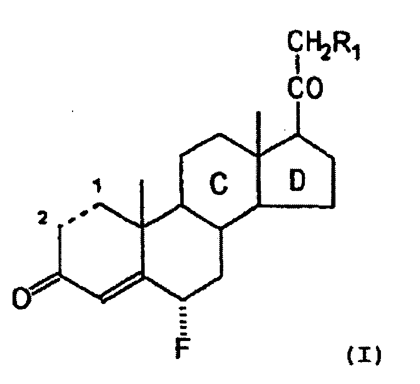 dans laquelle
la ligne de tirets entre les positions 1 et 2 représente une liaison simple ou double ;
R₁ est OH, OCOR₂, X, SO₃R₃ ou un groupe (R₇)(R₈)(R₉)SiO, où X est un halogène, et R₂ et R₃ sont des radicaux alkyle en C₁₋₆ ou phényle éventuellement substitués par un radical alkyle en C₁₋₄, et R₇, R₈ et R₉, identiques ou différents, sont des radicaux alkyle en C₁₋₆ ou phényle éventuellement substitués par un radical alkyle en C₁₋₄;
le cycle C du stéroïde est : 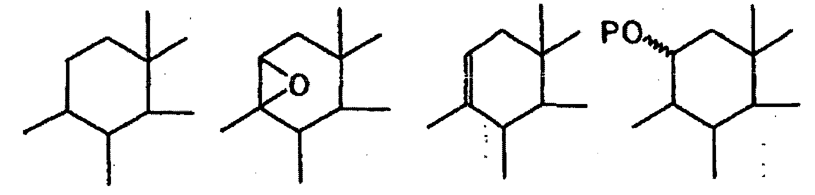
où :
P est un groupe protecteur du groupe hydroxyle ; et
le cycle D du stéroïde est : 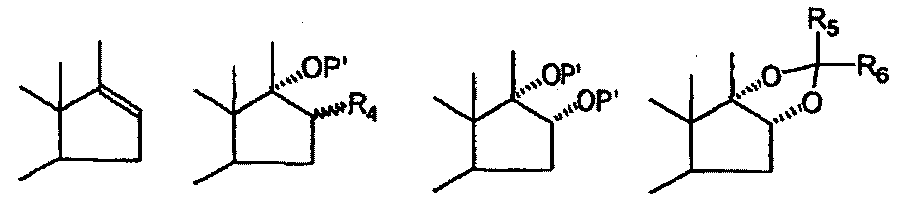
où :
R₄ est H ou CH₃ (configuration α ou β) ;
R₅ et R₆, identiques ou différents, sont des radicaux alkyle en C₁₋₄ ; et
chaque P' est indépendamment H, un groupe protecteur du groupe hydroxyle ou un groupe (R₇)(R₈)(R₉)Si-, où R₇, R₈ et R₉ ont les significations précédemment mentionnées ;
comprenant la réaction d'un silyloxy-prégna-3,5-diène 3-trisubstitué de formule générale (IV) : 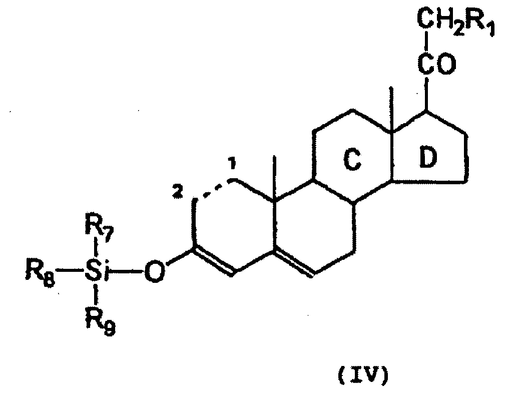
dans laquelle
la ligne de tirets entre les positions 1 et 2, R₁, R₇, R₈ et R₉, et les cycles C et D du stéroïde, ont les significations précédemment mentionnées,
avec un agent de fluoration choisi parmi :
(i) un N-fluorosulfonimide de formule générale (V)
R₁₀-O₂S-NH-SO₂-R₁₁ (V)
dans laquelle
R₁₀ et R₁₁, identiques ou différents, sont des radicaux alkyle en C₁₋₄ dont un ou plusieurs atomes d'hydrogène sont éventuellement substitués par des halogènes, ou phényle éventuellement substitués par un radical alkyle en C₁₋₄ ;
(ii) un N-fluorosulfonimide de formule générale (VI) : 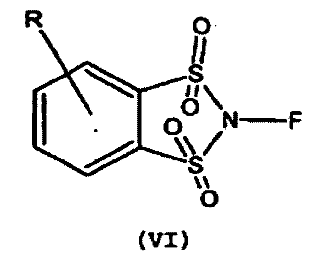 dans laquelle
R est un radical alkyle en C₁₋₆ ; et
(iii) un N-fluorosulfonamide de formule générale (VII) 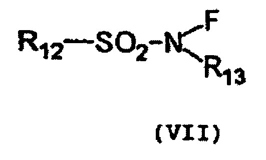 dans laquelle
R₁₂ est un radical phényle éventuellement substitué par un radical alkyle en C₁₋₄ ; et
R₁₃ est H, un radical alkyle en C₁₋₆ ou phényle éventuellement substitué par un radical alkyle en C₁₋₄.

2. Procédé selon la revendication 1, pour la production d'un composé de formule (I) dans lequel la ligne de tirets entre les positions 1 et 2 représente une double liaison.

3. Procédé selon la revendication 1, pour la production d'un composé de formule (I) dans lequel R₁ est le chlore ou un radical hydroxyle, acétate, pivalate, propionate ou mésylate.

4. Procédé selon la revendication 1, pour la production d'un composé de formule (I) présentant un groupe 9β,11β-époxy dans le cycle C, ou une double liaison entre les positions 9 et 11 du cycle C.

5. Procédé selon la revendication 1, pour la production d'un composé de formule (I) dans lequel R₄ est H, αCH₃ ou βCH₃.

6. Procédé selon la revendication 1, pour la production d'un composé de formule (I) contenant un groupe αOH en position 17.

7. Procédé selon la revendication 1, pour la production d'un composé de formule (I) dans lequel R₅ et R₆ sont simultanément des radicaux méthyle.

8. Procédé selon la revendication 1, pour la production d'un composé de formule (I) dans lequel la ligne de tirets entre les positions 1 et 2 représente une double liaison, R₁ est le chlore ou un radical hydroxyle, acétate, pivalate, propionate ou mésylate, a un groupe 9β,11β-époxy dans le cycle C, R₄ est H, αCH₃ ou βCH₃, et a un groupe αOH en position 17.

9. Procédé selon la revendication 1, pour la production d'un composé de formule (I) dans lequel la ligne de tirets entre les positions 1 et 2 représente une double liaison, R₁ est le chlore ou un radical hydroxyle, acétate, pivalate, propionate ou mésylate, a une double liaison entre les positions 9 et 11, R₄ est H, αCH₃ ou βCH₃, et a un groupe αOH en position 17.

10. Procédé selon la revendication 1, dans lequel la réaction entre le composé de formule (IV) et l'agent de fluoration choisi parmi les composés de formules (V), (VI) et (VII) est mise en oeuvre dans un solvant organique choisi parmi un solvant organique halogéné, un hydrocarbure aromatique, un éther, et l'acétonitrile.

11. Procédé selon la revendication 10, dans lequel ledit solvant organique halogéné est le chlorure de méthylène, le 1,2-dichloroéthane ou le chloroforme.

12. Procédé selon la revendication 1, dans lequel la réaction entre le composé de formule (IV) et l'agent de fluoration choisi parmi les composés de formules (V), (VI) et (VII) est mise en oeuvre en présence d'une base organique azotée.

13. Procédé selon la revendication 12, dans lequel la base organique azotée est le triazole, l'aminothiazole, l'imidazole ou la pyridine.

14. Procédé selon la revendication 1, dans lequel la réaction entre le composé de formule (IV) et l'agent de fluoration choisi parmi les composés de formules (V), (VI) et (VII) est mise en oeuvre à une température comprise entre -40°C et +20°C, de préférence entre -10°C et 0°C.

15. Composé de formule générale (IV): 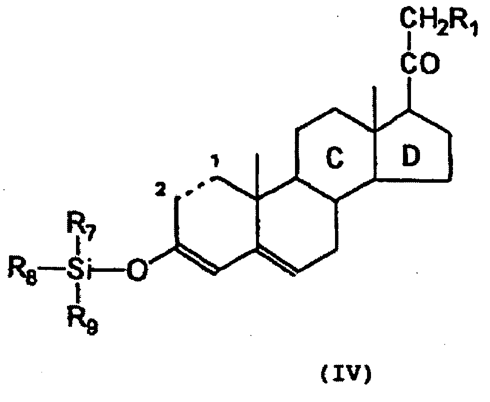 dans laquelle
la ligne de tirets entre les positions 1 et 2 représente une liaison simple ou double ;
R₁ est OH, OCOR₂, X, SO₃R₃ ou un groupe (R₇)(R₈)(R₉)SiO, où X est un halogène, et R₂ et R₃ sont des radicaux alkyle en C₁₋₆ ou phényle éventuellement substitués par un radical alkyle en C₁₋₄, et R₇, R₈ et R₉, identiques ou différents, sont des radicaux alkyle en C₁₋₆ ou phényle éventuellement substitués par un radical alkyle en C₁₋₄ ;
le cycle C du stéroïde est : 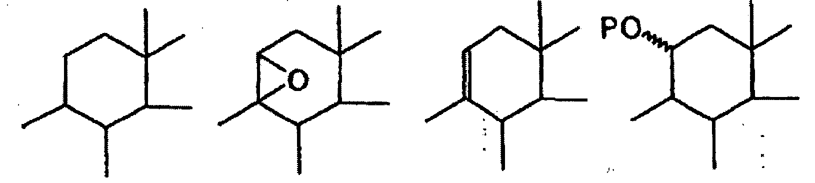
où :
P est un groupe protecteur du groupe hydroxyle ; et
le cycle D du stéroïde est : 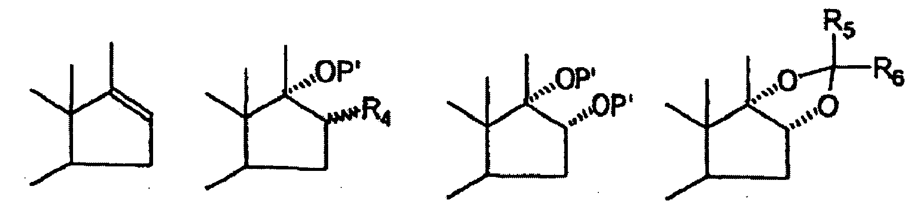
où :
R₄ est H ou CH₃ (configuration α ou β) ;
R₅ et R₆, identiques ou différents, sont des radicaux alkyle en C₁₋₄ ; et
chaque P' est indépendamment H, un groupe protecteur du groupe hydroxyle ou un groupe (R₇)(R₈)(R₉)Si-, où R₇, R₈ et R₉ ont les significations précédemment mentionnées.

16. Composé selon la revendication 15, dans lequel la ligne de tirets entre les positions 1 et 2 représente une double liaison.

17. Composé selon la revendication 15, dans lequel R₁ est un radical acétate, pivalate, propionate ou mésylate.

18. Composé selon la revendication 15, ayant un groupe 9β, 11β-époxy dans le cycle C ou une double liaison entre les positions 9 et 11 du cycle C.

19. Composé selon la revendication 15, dans lequel R₄ est H, αCH₃ ou βCH₃.

20. Composé selon la revendication 15, contenant un groupe αOH en position 17.

21. Composé selon la revendication 15, dans lequel R₅ et R₆ sont simultanément des radicaux méthyle.

22. Composé selon la revendication 15, dans lequel deux groupes choisis parmi R₇, R₈ et R₉ sont simultanément des radicaux méthyle et celui restant est le radical t-butyle, ou dans lequel R₇, R₈ et R₉ sont simultanément des radicaux isopropyle.

23. Composé selon la revendication 15, dans lequel la ligne de tirets entre les positions 1 et 2 représente une double liaison, R₁ est un radical acétate, pivalate, propionate ou mésylate, il a un groupe 9β, 11β-époxy dans le cycle C, R₄ est αCH₃ ou βCH₃, il a un groupe αOH en position 17, deux groupes choisis parmi R₇, R₈ et R₉ sont simultanément des radicaux méthyle et celui restant est le radical t-butyle, ou dans lequel R₇, R₈ et R₉ sont simultanément des radicaux isopropyle.

24. Composé selon la revendication 15, dans lequel la ligne de tirets entre les positions 1 et 2 représente une double liaison, R₁ est un radical acétate, pivalate, propionate ou mésylate, il a une double liaison entre les positions 9 et 11, R₄ est αCH₃ ou βCH₃, il a un groupe αOH en position 17, deux groupes choisis parmi R₇, R₈ et R₉ sont simultanément des radicaux méthyle et celui restant est le radical t-butyle, ou dans lequel R₇, R₈ et R₉ sont simultanément des radicaux isopropyle.

25. Composé selon la revendication 15, contenant un groupe (R₇)(R₉)(R₉)SiO en positions 16 et/ou 21.

26. Procédé pour obtenir un composé de formule (IV) selon l'une quelconque des revendications 15 à 25, comprenant la réaction d'un dérivé de prégnane de formule générale (II) : 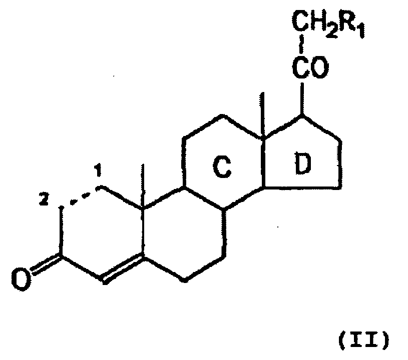 dans laquelle
la ligne de tirets entre les positions 1 et 2 représente une liaison simple ou double ;
R₁ est OH, OCOR₂, X, SO₃R₃ ou un groupe (R₇)(R₈)(R₉)SiO, où X est un halogène, et R₂ et R₃ sont des radicaux alkyle en C₁₋₆ ou phényle éventuellement substitués par un radical alkyle en C₁₋₄, et R₇, R₈ et R₉, identiques ou différents, sont des radicaux alkyle en C₁₋₆ ou phényle éventuellement substitués par un radical alkyle en C₁₋₄ ;
le cycle C du stéroïde est : 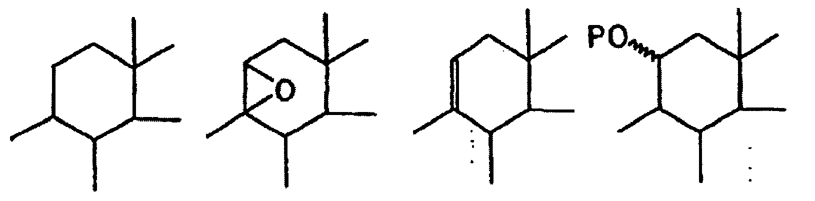
où :
P est un groupe protecteur du groupe hydroxyle ; et
le cycle D du stéroïde est : 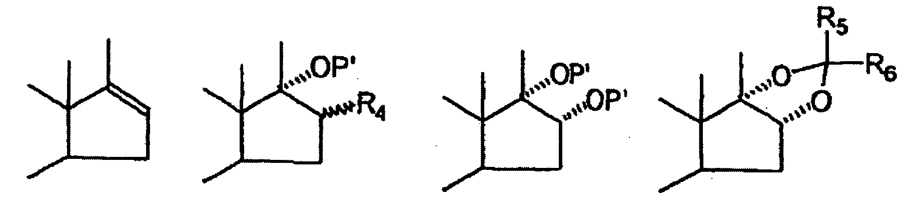
où :
R₄ est H ou CH₃ (configuration α ou β) ;
R₅ et R₆, identiques ou différents, sont des radicaux alkyle en C₁₋₄ ; et
chaque P' est indépendamment H, un groupe protecteur du groupe hydroxyle ou un groupe (R₇)(R₈)(R₉)Si-, où R₇, R₈ et R₉ ont les significations précédemment mentionnées ;
avec un silyltrifluorométhanesulfonate trisubstitué de formule générale (III) : 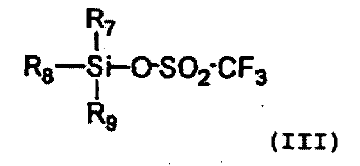
dans laquelle
R₇, R₈ et R₉ ont les significations précédemment mentionnées.

27. Procédé selon la revendication 26, dans lequel ledit composé de formule (III) est le trifluorométhanesulfonate de t-butyldiméthylsilyle ou le trifluorométhanesulfonate de triisopropylsilyle.

28. Procédé selon la revendication 26, dans lequel la réaction entre le composé de formule (II) et le composé de formule (III) est mise en oeuvre dans un solvant organique choisi parmi un solvant organique halogéné, un éther, et l'acétonitrile.

29. Procédé selon la revendication 28, dans lequel ledit solvant halogéné est le dichlorométhane ou le 1,2-dichloroéthane.

30. Procédé selon la revendication 26, dans lequel la réaction entre le composé de formule (II) et le composé de formule (III) est mise en oeuvre en présence d'une base organique azotée.

31. Procédé selon la revendication 30, dans lequel ladite base organique azotée est la diisopropyléthylamine, la triéthylamine, la lutidine ou la collidine.

32. Procédé selon la revendication 26, dans lequel la réaction entre le composé de formule (II) et le composé de formule (III) est mise en oeuvre à une température comprise entre -20°C et 25°C, de préférence entre -10°C et 0°C.

33. Procédé selon la revendication 26, dans lequel la réaction entre le composé de formule (II) et le composé de formule (III) est mise en oeuvre à un rapport molaire composé (III)/composé (II) égal ou supérieur à 2 pour qu'on obtienne le dérivé disilylé du composé de formule (IV), ou égal ou supérieur à 3 pour qu'on obtienne le dérivé trisilylé du composé de formule (IV).

34. Procédé selon la revendication 26, dans lequel le composé de formule (II) contient un groupe (R₇)(R₈)(R₉)Si-, où R₇, R₈ et R₉, identiques ou différents, sont des radicaux alkyle en C₁₋₆ ou phényle éventuellement substitués par un radical alkyle en C₁₋₄ en positions 16 et/ou 21, et la réaction entre ledit composé de formule (II) et le composé de formule (III) est mise en oeuvre à un rapport molaire convenable pour qu'on obtienne le dérivé disilylé ou le dérivé trisilylé du composé de formule (IV).

35. Procédé selon la revendication 1 pour la production de 6α-fluoroprégnane (I) : 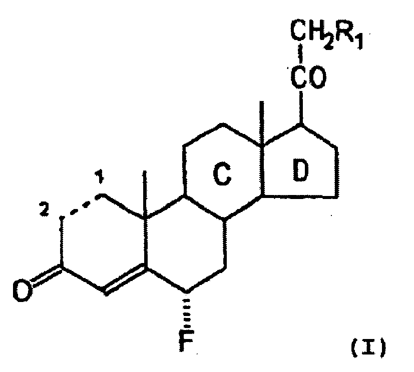 dans laquelle
la ligne de tirets entre les positions 1 et 2 représente une liaison simple ou double ;
R₁ est OH, OCOR₂, X, SO₃R₃ ou un groupe (R₇)(R₈)(R₉)SiO, où X est un halogène, et R₂ et R₃ sont des radicaux alkyle en C₁₋₆ ou phényle éventuellement substitués par un radical alkyle en C₁₋₄, et R₇, R₈ et R₉, identiques ou différents, sont des radicaux alkyle en C₁₋₆ ou phényle éventuellement substitués par un radical alkyle en C₁₋₄ ;
le cycle C du stéroïde est : 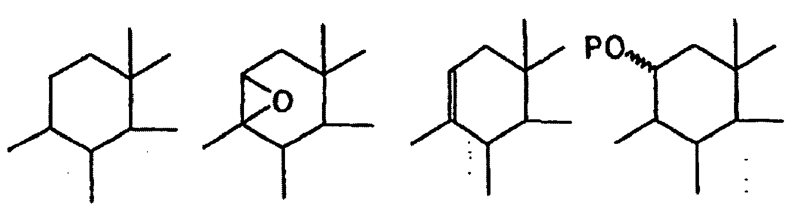
où :
P est un groupe protecteur du groupe hydroxyle ; et
le cycle D du stéroïde est : 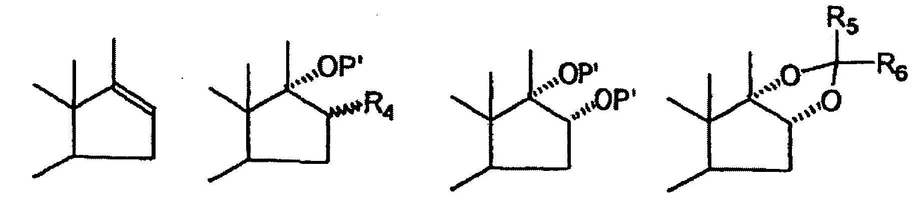
où :
R₄ est H ou CH₃ (configuration α ou β) ;
R₅ et R₆, identiques ou différents, sont des radicaux alkyle en C₁₋₄ ; et
chaque P' est indépendamment H, un groupe protecteur du groupe hydroxyle ou un groupe (R₇)(R₈)(R₉)Si-, où R₇, R₈ et R₉ ont les significations précédemment mentionnées ;
le procédé comprenant en outre la production du composé de formule (IV) par réaction d'un dérivé de prégnane de formule générale (II) : 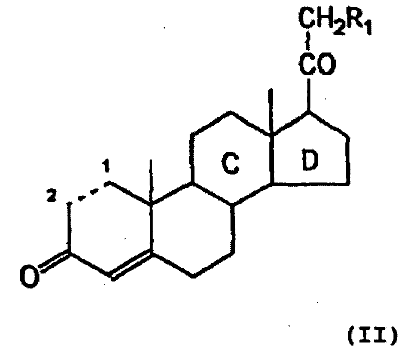
dans laquelle
la ligne de tirets entre les positions 1 et 2, R₁, et les cycles C et D ont les significations précédemment mentionnées,
avec un silyltrifluorométhanesulfonate trisubstitué de formule générale (III) : 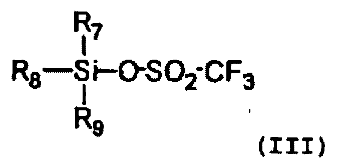
dans laquelle
R₇, R₈ et R₉ ont les significations précédemment mentionnées,
pour qu'on obtienne un composé de formule (IV) : 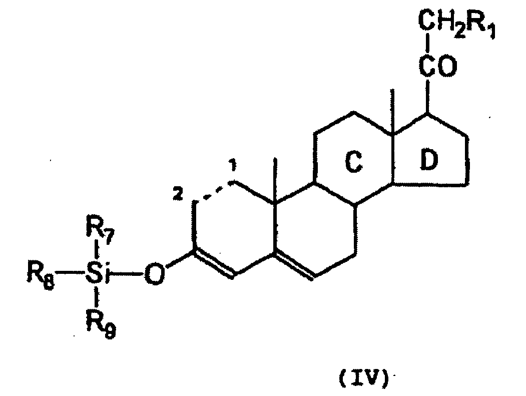
dans laquelle
la ligne de tirets entre les positions 1 et 2, R₁, R₇, R₈, R₉, et les cycles C et D ont les significations précédemment mentionnées.

36. Procédé selon la revendication 35, comprenant l'isolation du composé de formule (IV) formé par réaction du composé de formule (II) avec le composé de formule (III) avant sa réaction avec l'agent de fluoration.

37. Procédé selon la revendication 35, dans lequel la réaction du composé de formule (IV) avec le composé de formule (V), (VI) ou (VII) a lieu sans isolation du composé de formule (IV) formé par réaction du composé de formule (II) avec le composé de formule (III).

38. Procédé selon la revendication 37, comprenant l'élimination des contaminants solubles dans l'eau générés après la réaction du composé de formule (II) avec le composé de formule (III) pour la formation du composé de formule (IV) et avant la réaction de ce dernier avec le composé de formule (V), (VI) ou (VII).
